# EUROPEAN PATENT APPLICATION

(11) **EP 4 835 897 A1**
(43) Date of publication of application: **04.11.2026**
(21) Application number: 24913448.7
(22) Date of filing: 21.11.2024
(51) Int. Cl.: G16B 25/00, G16B 40/00, C12Q 1/6851, G16B 20/20, G16B 30/10

(54) **METHOD FOR DETECTING TARGET ANALYTE IN SAMPLE**

(30) Priority: 26.12.2023 KR 20230191631
(71) Applicant: Seegene, Inc., Seoul 05548 (KR)
(72) Inventor: CHO, Jung Hwan, Seoul 05544 (KR); HA, Yea Seong, Uiwang-si, Gyeonggi-do 16049 (KR); LEE, Han Bit, Seoul 05546 (KR); MIN, Il Jae, Seoul 05620 (KR); PARK, Seong Won, Seoul 04932 (KR)
(74) Representative: Engelhard, Markus
(86) International application number: PCT/KR2024/018483
(87) International publication number: WO 2025/143555

(57) **Abstract**

The present invention relates to a method for detecting a target analyte in a sample or a method for determining a quantification cycle (Cq) value for a target analyte in a sample. The present invention utilizes, for detection of the target analyte, an intersection point between a function representing an amplification curve for the target analyte and a first-order function connecting a first extreme point of an n-th order differential function or an n+1-th order differential function of the function representing the amplification curve and a second extreme point of the n+1-th order differential function, thereby providing superior quantitative accuracy and precision compared to conventional methods, even in the presence of inter-experiment variations in real-time PCR reactions.

## Description

### CROSS-REFERENCE TO RELATED APPLICATIONS

This patent application claims priority from Korean Patent Application No. 2023-0191631, filed on December 26, 2023 in the Korean Intellectual Property Office, the disclosure of which is incorporated herein by reference.

### FIELD OF THE INVENTION

The present invention relates to a method for detecting a target analyte in a sample or a method for determining a quantification cycle (Cq) value for a target analyte in a sample.

### DESCRIPTION OF THE RELATED ART

Nucleic acid amplification and detection have become one of the core techniques in modern molecular biology research. The polymerase chain reaction (PCR), which is the most widely used technique for nucleic acid amplification, involves repeated cycles of denaturation of double-stranded DNA, annealing of oligonucleotide primers to a DNA template, and extension of the primers by a DNA polymerase (Mullis et al., U.S. Patent Nos. 4,683,195; 4,683,202; and 4,800,159; Saiki et al., (1985) Science 230, 1350-1354).

Real-time PCR is a PCR-based technique for detecting a target nucleic acid in a sample in real time. In order to detect a specific target nucleic acid, real-time PCR uses a signal-generating means capable of producing a detectable fluorescent signal in proportion to the amount of the target nucleic acid. Generation of the fluorescent signal may be achieved by using an intercalator that generates a signal when intercalated between double-stranded DNA or an oligonucleotide comprising a fluorescent reporter and a quencher molecule. A fluorescent signal having an intensity proportional to the amount of the target molecule is detected at each amplification cycle and plotted against the amplification cycle, thereby obtaining an amplification curve or an amplification profile curve.

A PCR amplification curve typically has multiple phases, including a baseline phase in which fluorescence remains substantially constant, an exponential phase in which fluorescence nearly doubles at each cycle, and a plateau phase in which amplification gradually decreases.

The baseline phase refers to a region in which the fluorescent signal exhibits little change during the initial stages of PCR. In the baseline region, the level of PCR amplicons is insufficient for detection, and thus the signal detected in this region may be attributed to background signal including fluorescence signal originating from reaction reagents and measurement devices. The exponential phase represents an increase in the fluorescent signal proportional to an increase in amplification products. The plateau phase refers to a region in which the fluorescent signal exhibits little increase due to saturation of PCR amplicons and fluorescent signal levels.

In particular, real-time PCR has been widely used in many fields due to its sensitivity and specificity. For example, real-time PCR can be used for qualitative or quantitative analysis of a target nucleic acid through amplification. This is based on various analytical methods for detecting a target analyte in a sample using data sets obtained by real-time PCR developed to date.

One method determines whether a signal value in a data set reaches or exceeds a predetermined threshold value. This method requires collection of as many actual reaction data sets as possible in order to determine the threshold value.

As another method, U.S. Patent No. 6,303,305 discloses a method of determining a maximum point of an n-th derivative of an amplification curve and calculating an initial concentration of a nucleic acid in a sample from the maximum point, and U.S. Patent No. 6,503,720 discloses a method for quantifying a concentration of a nucleic acid in a sample by calculating a first-order, second-order, or n-th order differential function of an amplification signal, determining a cycle corresponding to a maximum point or a minimum point of the differential function, and calculating an initial concentration in the sample from the maximum point or the minimum point.

However, the above-described methods have a problem in that they may be relatively susceptible to experimental variations in experimental PCR reactions.

Accordingly, the present inventors have recognized the need to develop a technique capable of overcoming quantitative inaccuracies arising from experimental variations in real-time PCR reactions.

Throughout this application, various patents and publications are referenced and citations are provided in parentheses. The disclosure of these patents and publications in their entities are hereby incorporated by references into this application in order to more fully describe this invention and the state of the art to which this invention pertains.

### SUMMARY OF THE INVENTION

The present inventors have endeavored to develop a technique capable of reducing the effects of experimental variations in real-time PCR reactions and improving the accuracy of quantitative PCR. As a result, the present inventors, unlike conventional methods that detect a target analyte in a sample by using a maximum point or a minimum point of a first-order, second-order, or n-th order differential function of a function representing an amplification curve for the target analyte, have found that, when an intersection point between (i) a first-order function connecting a first extreme point of an n-th order differential function or an n+1-th order differential function of a function representing an amplification curve for the target analyte and a second extreme point of the n+1-th order differential function, and (ii) the function representing the amplification curve for the target analyte is used for detection of the target analyte, quantitative accuracy and precision are superior to those of conventional methods, even in the presence of inter-experiment variations in real-time PCR reactions, thereby completing the present invention.

Accordingly, it is an object of the present invention to provide a method for detecting a target analyte in a sample.

It is another object of the present invention to provide a method for determining a quantification cycle (Cq) value for a target analyte in a sample.

It is still another object of the present invention to provide a computer-readable storage medium containing instructions to configure a processor to perform the method of the present invention as described above.

Other objects and advantages of the present invention will become apparent from the detailed description to follow taken in conjugation with the appended claims and drawings.

### DETAILED DESCRIPTION OF THIS INVETNION

### I. Method for detecting a target analyte in a sample (first aspect)

In an aspect of the present invention, there is provided a method for detecting a target analyte in a sample, comprising:
(a) obtaining an S-shaped function representing a growth curve by an amplification reaction for a target analyte;
(b) obtaining an n-th order differential function and an n+1-th order differential function from the S-shaped function, wherein n is a natural number;
(c) obtaining a first order function connecting (i) a first extreme point of the n-th order differential function or the n+1-th order differential function and (ii) a second extreme point of the n+1-th order differential function; and
(d) detecting the target analyte in the sample by using an intersection point between the S-shaped function of step (a) and the first order function of step (c).

The present inventors have endeavored to develop a technique capable of reducing the effects of experimental variations in real-time PCR reactions and improving the accuracy of quantitative PCR. As a result, unlike conventional methods that detect a target analyte in a sample by using a maximum point or a minimum point of a first-order, second-order, or n-th order differential function of a function representing an amplification curve for the target analyte, the present inventors have confirmed that, when an intersection point between (i) a first-order function connecting a first extreme point of an n-th order differential function or an n+1-th order differential function of a function representing an amplification curve for the target analyte and a second extreme point of the n+1-th order differential function, and (ii) the function representing the amplification curve for the target analyte is used for detection of the target analyte, quantitative accuracy and precision are superior to those of the conventional methods, despite inter-experiment variations in real-time PCR reactions.

According to one embodiment, the method is a computer-implemented method.

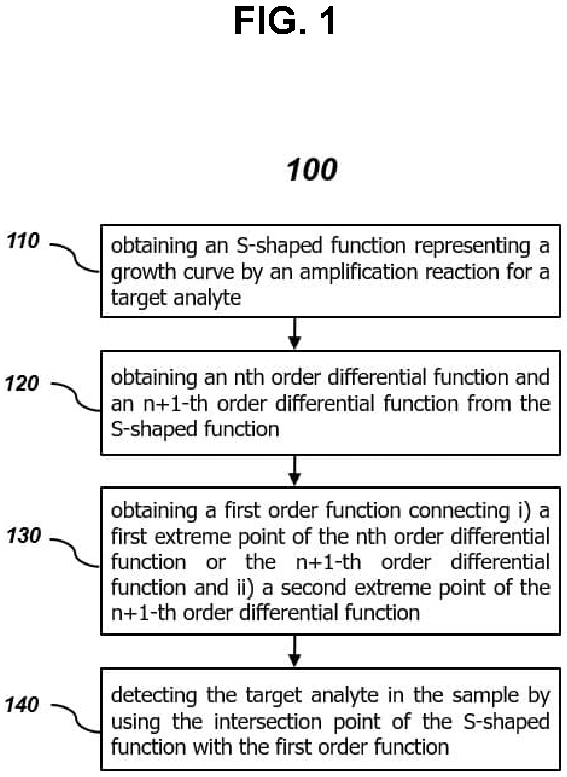

FIG. 1 is a flowchart illustrating processes for performing the method of the present invention according to one embodiment. The method of the present invention will now be described with reference to FIG. 1.

### Step (a): Obtaining an S-shaped function representing a growth curve (110)

First, the method of the present invention comprises step (a) of obtaining an S-shaped function representing a growth curve by an amplification reaction for a target analyte.

The term "sample" as used herein refers to any material comprising, or suspected of comprising, a nucleic acid of interest, or any material comprising a nucleic acid itself that comprises, or is suspected of comprising, a target nucleic acid sequence of interest. Specifically, the sample includes biological samples (*e.g.,* cells, tissues, and bodily fluids from a biological source) and non-biological samples (*e.g.,* food, water, and soil). Biological samples include, without limitation, viruses, bacteria, tissues, cells, blood, serum, plasma, lymph, sputum, swabs, aspirates, bronchial lavage fluid, milk, urine, feces, ocular fluid, saliva, semen, brain extracts, cerebrospinal fluid (CSF), extracts of appendix, spleen, and tonsil tissues, ascites fluid, and amniotic fluid. The sample may further include naturally occurring nucleic acid molecules isolated from a biological source as well as synthetic nucleic acid molecules.

As used herein, the term "target analyte" may include a variety of substances, including biological and non-biological substances, such as chemical substances. Specifically, the target analyte may include biological materials such as nucleic acid molecules (*e.g.,* DNA and RNA), proteins, peptides, carbohydrates, lipids, amino acids, biological compounds, hormones, antibodies, antigens, metabolites, and cells. More specifically, the target analyte may include a nucleic acid molecule. The target analyte is present in the sample.

As used herein, the terms "target nucleic acid," "target nucleic acid sequence," or "target sequence" refer to a nucleic acid sequence of interest for analysis or detection. The target nucleic acid sequence includes not only single-stranded sequences but also double-stranded sequences. The target nucleic acid sequence includes sequences initially present in the sample as well as sequences newly generated during the reaction.

The target nucleic acid sequence includes any DNA (gDNA and cDNA), RNA molecules, and hybrids thereof (chimeric nucleic acids). The sequence may be in a double-stranded or single-stranded form. When a nucleic acid is double-stranded as a starting material, it is preferable to convert the double-stranded nucleic acid into a single-stranded or partially single-stranded form. Known methods for separating strands include, without limitation, heating, treatment with alkali, formamide, urea, or glyoxal, enzymatic methods (*e.g.,* helicase activity), and binding proteins. For example, strand separation may be achieved by heating at a temperature of from 80°C to 105°C. General methods for accomplishing such treatments are disclosed in the literature (Joseph Sambrook et al., Molecular Cloning: A Laboratory Manual, Cold Spring Harbor Laboratory Press, Cold Spring Harbor, N.Y. (2001)).

The target nucleic acid sequence includes any naturally occurring prokaryotic nucleic acid, eukaryotic nucleic acid (*e.g.,* protozoa and parasites, fungi, yeast, higher plants, lower animals, and higher animals such as mammals and humans), viral nucleic acid (*e.g.,* herpes viruses, HIV, influenza viruses, Epstein-Barr virus, hepatitis viruses, poliovirus, etc.), or viroid nucleic acid. The nucleic acid molecule may also be any nucleic acid molecule that is produced or can be produced recombinantly, or chemically synthesized or capable of being synthesized. Accordingly, the target nucleic acid sequence may or may not be found in nature.

The target nucleic acid sequence should not be construed as being limited to sequences known at a given time point or sequences available from a given time point, but rather should be construed as including sequences that are available or may become known at any present or future time point. That is, the target nucleic acid sequence may or may not be known at the time of practicing the method of the present invention. For an unknown target nucleic acid sequence, the sequence may be determined by one of the conventional sequencing methods prior to practicing the present invention.

When the target analyte is a target nucleic acid molecule, the sample may undergo a nucleic acid extraction process. The nucleic acid extraction process may vary depending on the type of the sample. When the extracted nucleic acid is RNA, a reverse transcription process for synthesizing cDNA from the extracted RNA is additionally performed (see Sambrook, J. et al., Molecular Cloning: A Laboratory Manual, 3rd ed., Cold Spring Harbor Press (2001)).

According to one embodiment, the target nucleic acid sequence includes a nucleotide variation.

As used herein, the term "nucleotide variation" refers to substitution, deletion, or insertion of one or more nucleotides at a specific position in a DNA sequence among contiguous DNA segments having similar sequences. Such contiguous DNA segments may include a gene or other regions of a chromosome. Such nucleotide variations may be mutants or polymorphic allele variations. For example, nucleotide variations detected in the present invention include single nucleotide polymorphisms (SNPs), mutations, deletions, insertions, substitutions, and translocations. Exemplary nucleotide variations include various variations in the human genome (*e.g.,* variations in methylenetetrahydrofolate reductase (MTHFR)), variations associated with drug resistance of pathogens, and oncogenic variations. As used herein, the term "nucleotide variation" includes any variation at a specific position in a nucleic acid sequence. That is, the term "nucleotide variation" includes a wild-type sequence and any mutant form thereof at a specific position in a nucleic acid sequence.

Various methods for amplifying a target nucleic acid molecule are known in the art, including, without limitation, polymerase chain reaction (PCR), ligase chain reaction (LCR) (see Wiedmann M. et al., "Ligase chain reaction (LCR)-overview and applications," PCR Methods and Applications, 3(4): S51-64 (1994)), gap-filling LCR (GLCR) (see WO 90/01069, EP 0439182, and WO 93/00447), Q-beta replicase amplification (Q-beta) (see Cahill P. et al., Clin. Chem., 37(9):1482-1485 (1991); U.S. Patent No. 5,556,751), strand displacement amplification (SDA) (see G. T. Walker et al., Nucleic Acids Research, 20(7):1691-1696 (1992); EP 0497272), nucleic acid sequence-based amplification (NASBA) (see Compton, J., Nature, 350(6313):912 (1991)), transcription-mediated amplification (TMA) (see Hofmann W. P. et al., Journal of Clinical Virology, 32(4):289-293 (2005); U.S. Patent No. 5,888,779), or rolling circle amplification (RCA) (see Hutchison C. A. et al., Proceedings of the National Academy of Sciences USA, 102:17332-17336 (2005)).

The S-shaped function used in the method of the present invention may be a function known in the art that approximates a growth curve for a target analyte, particularly an amplification curve, and more particularly a real-time PCR curve.

As used herein, the term "growth curve" includes (1) a curve composed of a raw data set of nucleic acid amplification, (2) a modification of (1), specifically including (i) baselining and (ii) smoothing, and (3) a derivation of (1), specifically including (i) a transformation into an amplification curve after n-th order differentiation and (ii) a transformation into an amplification curve after n-th order differencing (*e.g.,* slope regression).

As used herein, the term "baselining" refers to a process of processing a data set by subtracting a value corresponding to a baseline from a signal value at each cycle of the data set, thereby obtaining a baseline-subtracted data set.

The baseline-subtracted data set may be obtained by various methods known in the art (U.S. Patent No. 8,560,247 and WO 2016/052991). In addition, a data set including a data set before normalization and a data set after normalization may be baselined using a quadratic function having an axis of symmetry. Baselining a data set using a quadratic function means correcting the data set by subtracting the quadratic function from the data set.

As used herein, the term "smoothing" refers to processing or refining a data set according to a predetermined algorithm in order to remove or minimize noise in the data set. Through smoothing of the data set, the visual representation of the data set is improved.

In data analysis, smoothing of a data set refers to generating an approximate data set that follows the main pattern of the data set. Smoothing of the data set removes noise, other fine structures, or abrupt phenomena. Through the smoothing process, signals of data points are processed, whereby characteristics of individual data points are reduced and signal differences between adjacent data points are reduced. This facilitates easier recognition of information regarding macroscopic changes in signals of the data set.

According to one embodiment of the present invention, the S-shaped function is selected from the group consisting of a sigmoid function, a logistic function, a Gompertz function, a Chapman function, and a Richard function.

According to a more specific embodiment, the sigmoid function is represented by Equation 1 below: $f \left(x\right) = {α}_{1} + \frac{{α}_{2} - {α}_{1}}{1 + {10}^{{α}_{4} \left({α}_{3}-x\right)}}$ Equation 1

In the above equation, α1, α2, α3, and α4 are parameters determined by fitting the sigmoid function to a selected portion of a data set, and x is a cycle number.

In the above equation, α1 may correspond to a background value in the curve represented by the function; α2 may correspond to a maximum value in the curve represented by the function; α3 may correspond to a cycle number having a midpoint value in the curve represented by the function; and α4 may correspond to a slope of the curve represented by the function.

The sigmoid function is a special case of a logistic function and has a characteristic S-shaped curve or sigmoid curve.

According to a more specific embodiment, the logistic function is represented by Equation 2 below: $f \left(x\right) = {α}_{1} + \frac{{α}_{2} - {α}_{1}}{1 + {\left(\frac{x}{{α}_{3}}\right)}^{{α}_{4}}}$ Equation 2

In the above equation, α1, α2, α3, and α4 are parameters determined by fitting the logistic function to a selected portion of a data set, and x is a cycle number.

In the above equation, α1 may correspond to a background value in the curve represented by the function; α2 may correspond to a maximum value in the curve represented by the function; α3 may correspond to a cycle number having a midpoint value in the curve represented by the function; and α4 may correspond to a slope of the curve represented by the function.

The logistic function is a function having a characteristic S-shaped curve or sigmoid curve.

According to a more specific embodiment, the Gompertz function is represented by Equation 3 below: $f \left(x\right) = {α}_{1} + \left({α}_{2}-{α}_{1}\right) {10}^{- {e}^{\frac{\left({α}_{3}-x\right)}{{α}_{4}}}}$ Equation 3

In the above equation, α1, α2, α3, and α4 are parameters determined by fitting the Gompertz function to a selected portion of a data set, and x is a cycle number.

In the above equation, α1 may correspond to a background value in the curve represented by the function; α2 may correspond to a maximum value in the curve represented by the function; α3 may correspond to a cycle number having a midpoint value in the curve represented by the function; and α4 may correspond to a slope of the curve represented by the function.

The Gompertz function is a type of sigmoid function and has a curve shape that increases steeply and then gradually approaches a horizontal asymptote.

According to a more specific embodiment, the Chapman function is represented by Equation 4 below: $f \left(x\right) = {α}_{1} + \left({α}_{2}-{α}_{1}\right) {\left(1-{10}^{- {α}_{4} x}\right)}^{c}$ Equation 4

In the above equation, α1, α2, α3, and α4 are parameters determined by fitting the Chapman function to a selected portion of a data set, and x is a cycle number.

In the above equation, α1 may correspond to a background value in the curve represented by the function; α2 may correspond to a maximum value in the curve represented by the function; α3 may correspond to a cycle number having a midpoint value in the curve represented by the function; and α4 may correspond to a slope of the curve represented by the function.

The Chapman function is a type of sigmoid function.

According to a more specific embodiment, the Richard function is represented by Equation 5 below: $f \left(x\right) = \frac{{F}_{max}}{{\left[1+{e}^{\left(-\frac{1}{b}\left(x-c\right)\right)}\right]}^{d}} + {F}_{b}$ Equation 5

In the above equation, x is a cycle number; F(x) is a reaction fluorescence at the cycle; Fmax is a maximum reaction fluorescence; b is a slope of the curve; c is a fractional cycle at which the reaction fluorescence is reached; d is a Richard coefficient; and Fb is background fluorescence.

Additional descriptions of the S-shaped function may be found in the literature (Michele Guescini et al., BMC Bioinformatics, 2008, 9:326).

According to one embodiment, the step of obtaining the S-shaped function of step (a) comprises the following steps:
(a-1) obtaining a first data set representing the growth curve by the amplification reaction for the target analyte, wherein the first data set includes a plurality of data points, each having a cycle number and a signal value at the cycle number;
(a-2) calculating a slope value at each cycle number for the first data set to obtain a second data set, wherein the second data set includes a plurality of data points, each having a cycle number and a slope value at the cycle number; and
(a-3) calculating the S-shaped function that approximates a selected portion of the second data set.

### (a-1) Obtaining a first data set for a target analyte

According to the present embodiment, in step (a-1), a first data set representing the growth curve by the amplification reaction for the target analyte is obtained. The first data set includes a plurality of data points, each having a cycle number and a signal value at the cycle number.

The first data set may be interchangeably used with an "amplification data set."

According to one embodiment, the first data set is obtained by a signal-generating process.

As used herein, the term "signal-generating process" refers to any process capable of generating a signal depending on characteristics of a target analyte in a sample, that is, activity, amount, or presence (or absence) of the target analyte, particularly presence (or absence) of the analyte in the sample. In the present application, the signal-generating process includes biological reactions and chemical reactions. Such biological reactions include genetic analyses such as PCR, real-time PCR, and microarray analysis, immunological analyses, and bacterial growth analyses. According to one embodiment, the signal-generating process includes analyzing generation, change, or destruction of a chemical substance.

The signal-generating process is accompanied by a signal change. The signal change may serve as an indicator that qualitatively or quantitatively represents presence or absence of a target nucleic acid sequence.

Details of the "signal-generating process" are disclosed in WO 2015/147412 filed by the present applicant, and the teachings thereof are incorporated herein by reference in their entirety.

According to one embodiment, the signal-generating process is an amplification process, particularly a signal amplification process.

According to one embodiment, the signal-generating process is a process with or without amplification of a target analyte.

Specifically, the signal-generating process is a process accompanied by amplification of a target nucleic acid molecule. More specifically, the signal-generating process is a process accompanied by amplification of a target nucleic acid molecule and capable of increasing or decreasing a signal upon amplification of the target nucleic acid molecule, particularly capable of increasing the signal.

As used herein, the term "signal generation" includes appearance or disappearance of a signal, and increase or decrease of a signal.

Specifically, the term "signal generation" refers to an increase in a signal.

The signal-generating process may be performed according to various methods known to those skilled in the art. Examples of such methods may be found in the description of signal-generating means below.

According to one embodiment, the signal-generating process may be performed as a process involving signal amplification together with target amplification.

According to one embodiment, as a signal-generating process, an amplification reaction may be performed in a manner in which a signal is amplified simultaneously with amplification of a target nucleic acid molecule (e.g., real-time PCR). Alternatively, a signal amplification reaction may be performed in a manner in which a signal is amplified without amplification of a target nucleic acid molecule (*e.g.,* the CPT method (Duck P. et al., Biotechniques, 9:142-148 (1990)) or the Invader assay (U.S. Patent Nos. 6,358,691 and 6,194,149)).

As used herein, the term "signal" refers to a measurable output. As used herein, the term "signal value" refers to an expression quantitatively representing a signal.

Signal intensity or signal change may serve as an indicator that qualitatively or quantitatively represents presence or absence of an analyte (target nucleic acid sequence).

Examples of useful indicators include fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectance, transmittance, and absorbance. Signal changes may include not only an increase in signal but also a decrease in signal. According to one embodiment, the signal-generating process is a process for amplifying a signal value.

The signal may include various signal characteristics obtained from signal detection, such as signal intensity (*e.g.,* relative fluorescence unit (RFU) values, or RFU values at a specific cycle, a selected cycle, or an end point when performing an amplification reaction), a shape (or pattern) of signal change, or a Ct value, or values obtained by mathematically processing the above characteristics.

According to one embodiment, the term "signal" includes not only a signal itself obtained at a detection temperature but also a modified signal obtained by mathematically processing the signal.

According to one embodiment, when an amplification curve is obtained by real-time PCR, various signal values (or characteristics) may be selected from the amplification curve and used to determine presence of a target (*e.g.,* intensity, Ct value, Cq value, or amplification curve data).

A signal (particularly, signal intensity) may vary depending not only on a detection temperature but also on a signal-generating means used.

As used herein, the term "signal-generating means" refers to any material used to generate a signal indicating characteristics of a target analyte to be analyzed, more specifically presence or absence of the target analyte.

As used herein, the term "signal-generating means" refers to any material used to generate a signal indicating presence of a target nucleic acid sequence, and includes, for example, oligonucleotides, labels, and enzymes. Alternatively, as used herein, the term "signal-generating means" may be used to refer to any method that uses a material for signal generation.

Various signal-generating means are known to those skilled in the art. The signal-generating means include a label itself and an oligonucleotide having a label. The label may include a fluorescent label, a luminescent label, a chemiluminescent label, an electrochemical label, and a metallic label. The label itself, for example an intercalating dye, may serve as a signal-generating means. Alternatively, an interactive dual label including a single label or a donor molecule and an acceptor molecule bound to at least one oligonucleotide may be used as a signal-generating means. The signal-generating means may include additional components for generating a signal, such as nucleic acid cleavage enzymes (*e.g.,* a 5'-nuclease and a 3'-nuclease).

The signal-generating means may be classified according to their signal-generating mechanisms as follows:
a. signal-generating means that generate a signal by formation of a duplex between a target nucleic acid sequence and a detection oligonucleotide that specifically hybridizes to the target nucleic acid sequence.

As used herein, the term "detection oligonucleotide" refers to an oligonucleotide involved in generation of a signal to be detected. According to one embodiment, the detection oligonucleotide includes an oligonucleotide that is involved in actual signal generation. For example, signal generation may depend on hybridization or non-hybridization of the detection oligonucleotide with another oligonucleotide (*e.g.,* an oligonucleotide comprising a nucleotide sequence complementary to a target nucleic acid sequence or to the detection oligonucleotide). According to one embodiment, the detection oligonucleotide includes at least one label.

Signal generation by formation of a duplex between a target nucleic acid sequence and a detection oligonucleotide may be achieved by various methods, including the Scorpion method (Whitcombe et al., Nature Biotechnology, 17:804-807 (1999)), the Sunrise or Amplifluor method (Nazarenko et al., Nucleic Acids Research, 25(12):2516-2521 (1997); and U.S. Patent No. 6,117,635), the Lux method (U.S. Patent No. 7,537,886), the Plexor method (Sherrill C. B. et al., Journal of the American Chemical Society, 126:4550-4556 (2004)), the molecular beacon method (Tyagi et al., Nature Biotechnology, Vol. 14, March 1996), the Hybeacon method (French D. J. et al., Molecular and Cellular Probes, 15(6):363-374 (2001)), the adjacent hybridization probe method (Bernard P. S. et al., Analytical Biochemistry, 273:221 (1999)), and the LNA method (U.S. Patent No. 6,977,295).

b. signal-generating means that generate a signal by formation of a duplex in a manner dependent on cleavage of a mediation oligonucleotide specifically hybridized to a target nucleic acid sequence.

As used herein, the term "mediation oligonucleotide" refers to an oligonucleotide that mediates formation of a duplex that does not include the target nucleic acid sequence.

According to one embodiment, cleavage of the mediation oligonucleotide itself does not generate a signal, and fragments generated by the cleavage participate in a subsequent reaction for signal generation.

Signal generation by a duplex formed in a manner dependent on cleavage of the mediation oligonucleotide may be achieved by various known methods, including the PTOCE (PTO cleavage and extension) method (WO 2012/096523), the PCE-SH (PTO Cleavage and Extension-Dependent Signaling Oligonucleotide Hybridization) method (WO 2013/115442), and the PCE-NH (PTO Cleavage and Extension-Dependent Non-Hybridization) method (WO 2014/104818).

c. signal-generating means that generate a signal by cleavage of a detection oligonucleotide after the detection oligonucleotide is hybridized to a target nucleic acid sequence.

Signal generation by cleavage of the detection oligonucleotide after the detection oligonucleotide is hybridized to the target nucleic acid sequence may be achieved by various methods, including the TaqMan probe method (U.S. Patent Nos. 5,210,015 and 5,538,848).

d. signal-generating means that generate a signal by cleavage of a detection oligonucleotide in a manner dependent on cleavage of a mediation oligonucleotide specifically hybridized to a target nucleic acid sequence.

According to one embodiment, when a mediation oligonucleotide hybridized to a target nucleic acid sequence is cleaved to release fragments, the fragments specifically hybridize to a detection oligonucleotide and induce cleavage of the detection oligonucleotide.

Signal generation by cleavage of a detection oligonucleotide in a manner dependent on cleavage of the mediation oligonucleotide may be achieved by various methods, including the Invader assay (U.S. Patent No. 5,691,142), the PCEC (PTO Cleavage and Extension-Dependent Cleavage) method (WO 2012/134195), and the method described in U.S. Patent No. 7,309,573.

As used herein, the term "signal amplification reaction" refers to a reaction that increases or decreases a signal generated by a signal-generating means.

According to one embodiment, the signal amplification reaction refers to an increase (or amplification) of a signal generated in dependence on presence of a target analyte using the signal-generating means. The signal amplification reaction may or may not be accompanied by amplification of the target analyte (*e.g.,* a target nucleic acid molecule). More specifically, the signal amplification reaction refers to signal amplification accompanied by amplification of the target analyte.

A first data set obtained by the signal amplification reaction includes cycle numbers.

As used herein, the term "cycle number" or "cycle" refers to a unit of change in conditions in a plurality of measurements accompanied by changes in conditions. For example, the changes in conditions may include changes in temperature, reaction time, number of reactions, concentration, pH, and/or number of replications of a target nucleic acid molecule sequence. Accordingly, a cycle may include a time cycle or a process cycle, a unit operation cycle, and a replication cycle.

As an example, when investigating enzyme kinetics, a reaction rate of an enzyme is measured several times while regularly increasing a concentration of a substrate. In this reaction, an increase in the substrate concentration may correspond to a change in conditions, and a unit of increase in the substrate concentration may correspond to a cycle.

As another example, in isothermal amplification, a sample is measured several times during a reaction time under isothermal conditions, and the reaction time may correspond to a change in conditions, while a unit of reaction time may correspond to a cycle. For example, when reaction times at intervals of 5 minutes, such as 5, 10, and 15 minutes, are set as respective reaction times, cycles may be expressed as a 5-minute cycle, a 10-minute cycle, a 15-minute cycle, and the like. Alternatively, when a 5-minute interval is regarded as one unit, the 5-minute cycle may be expressed as a first cycle, the 10-minute cycle as a second cycle, the 15-minute cycle as a third cycle, and the like.

As yet another example, in melting analysis or hybridization analysis, a change in a signal may be measured while changing a temperature within a predetermined temperature range, and the temperature may correspond to a change in conditions, while a temperature unit (*e.g.,* a measurement temperature) may correspond to a cycle. For example, when intervals of 0.5°C, such as 40°C, 40.5°C, 50°C, and 50.5°C, are set as respective measurement temperatures, cycles may be expressed as a 40°C cycle, a 40.5°C cycle, a 50°C cycle, a 50.5°C cycle, and the like. Alternatively, when a 0.5°C interval is regarded as one unit, the 40°C cycle may be expressed as a first cycle, the 40.5°C cycle as a second cycle, the 50°C cycle as a third cycle, and the like.

Specifically, when a series of reactions is repeated or reactions are repeated at regular time intervals, the term "cycle" refers to one unit of the repetition.

For example, in polymerase chain reaction (PCR), a cycle refers to a reaction unit including denaturation of a target molecule, annealing (hybridization) between the target molecule and a primer, and extension of the primer. An increase in repetitions of the reaction may correspond to a change in conditions, and a unit of the repetition may correspond to a cycle.

A first data set obtained by an amplification reaction includes a plurality of data points, and each data point includes a cycle number and a signal value at the cycle number.

As used herein, the term "signal value" refers to a numerical value of a signal level (*e.g.,* signal intensity) that is actually measured at each cycle of a signal-generating process, or a modification thereof. The modification may include a value obtained by mathematically processing the measured signal value. Examples of mathematically processed values of measured signal values (*i.e.,* signal values of a raw data set) include values obtained by addition, subtraction, multiplication, or division, or logarithmic values.

As used herein, the term "data point" refers to a coordinate value including a cycle and a signal value at the cycle. As used herein, the term "data" refers to all information constituting a data set. For example, each of a cycle and a signal value of an amplification reaction constitutes data.

Data points obtained from a signal-generating reaction, particularly a signal amplification reaction, may be plotted as coordinate values in a Cartesian coordinate system. In the Cartesian coordinate system, an X-axis represents cycles of the amplification reaction, and a Y-axis represents signal values measured at each cycle or modifications thereof.

As used herein, the term "data set" refers to a set of data points. For example, the data set may include a raw data set that is a set of data points obtained directly from an amplification reaction using a signal-generating means. Alternatively, the data set may be a modified data set obtained by modifying a data set including a set of data points obtained directly from a signal-generating process. The data set may include all or a part of a set of data points obtained from the signal-generating process. The data set includes a plurality of data points. The data set may include at least two data points. Specifically, the number of data points in the data set may be at least 2, 3, 4, 5, 10, or 20. In addition, the number of data points in the data set may be 1,000 or less. Specifically, the number of data points in the data set may be 1,000, 500, 300, 200, 100, 90, 80, 70, or 60 or less. The number of data points in the data set may be from 3 to 1,000. More specifically, the number of data points may be from 3 to 1,000, 10 to 500, 1 to 100, 20 to 100, 20 to 80, 20 to 70, or 20 to 60. According to one embodiment, the number of data points in the data set may be from 20 to 60.

According to one embodiment, the first data set may be obtained by processing a plurality of data sets. When two target analytes are detected in a single reaction, data sets for each of the two target analytes may be provided by processing a data set obtained by the single reaction. For example, the data sets for each of the two target analytes may be provided by obtaining two data sets by measuring signals at two different detection temperatures and processing the two data sets.

The first data set may be plotted to obtain an amplification curve.

According to one embodiment, the amplification curve is an amplification curve obtained by an amplification reaction of a target analyte, specifically a target nucleic acid molecule.

According to a more specific embodiment of the present invention, the first data set of step (a-1) may be a raw data set, a mathematically modified data set of the raw data set, a normalized data set of the raw data set, or a normalized data set of the mathematically modified data set.

According to an even more specific embodiment, the first data set of step (a-1) is a raw data set.

As used herein, the term "raw data set" refers to a data set including signals obtained directly from a signal-generating reaction. For example, the raw data set includes a set of signals which are obtained from a signal receiving device, have undergone basic signal processing at a data acquisition device and are subsequently provided to a signal analysis step.

According to an even more specific embodiment, the first data set of step (a-1) is a mathematically modified data set of a raw data set.

As used herein, the term "mathematically processed data set" refers to a data set transformed from a raw data set through mathematical processing. The mathematically processed data set may be a baseline-subtracted data set obtained by removing a background signal value from the raw data set. The baseline-subtracted data set may be obtained by various methods known in the art (*e.g.,* U.S. Patent No. 8,560,247).

According to a more specific embodiment, the first data set of step (a-1) is a normalized data set of a raw data set or a normalized data set of a mathematically modified data set.

As used herein, the term "normalization" refers to a process for reducing or eliminating signal variations among data sets for a plurality of reactions. As used herein, the terms "correction" or "adjustment" refer to correcting or modifying data of a data set, particularly signal values, for analysis purposes. Normalization is an example of correction.

According to one embodiment, the normalized data set may be provided by a method comprising the following steps:
(i) providing a normalization coefficient for calibrating the raw data set or the mathematically modified data set of the raw data set, wherein the data set is provided from a signal generated by a signal-generating reaction using a signal-generating means, wherein the data set includes a plurality of data points each having a cycle number and a signal value at the cycle number, wherein the normalization coefficient is provided using a reference value, a reference cycle, and the data set, wherein the reference value is an arbitrarily determined value, and wherein the normalization coefficient is provided by defining a relationship between the signal value at a cycle of the data set corresponding to the reference cycle and the reference value; and
(ii) applying the normalization coefficient to the signal values of the data set to obtain calibrated signal values, thereby providing the normalized data set.

The reference cycle is a cycle selected to determine a specific signal value used to provide the normalization coefficient having the reference value. The reference cycle used to provide the normalization coefficient may be arbitrarily selected from cycles of the data set.

According to one embodiment, the reference cycle may be selected from cycles within a baseline region.

A background signal is a signal generated not by the target analyte itself in a sample or by the target analyte present in a sample, but by the signal-generating means itself that does not involve the target analyte. The baseline region is a region in which little or no signal attributable to the target analyte is generated and only the background signal is generated.

The baseline region refers to an initial stage of a signal-generating process before signal amplification is sufficiently detectable. Specifically, the reference cycle may be determined from cycles within the baseline region, such as cycles 1 to 30, 2 to 30, 2 to 20, 2 to 15, 2 to 10, 2 to 8, 3 to 30, 3 to 20, 3 to 15, 3 to 10, 3 to 9, 3 to 8, 4 to 8, or 5 to 8.

A reference value is a value used to provide the normalization coefficient. The reference value of the present invention refers to an arbitrary value applied to a reference cycle number for correction of signal values of a data set. The reference value may be an arbitrarily determined value. Preferably, the reference value is an arbitrarily determined real number other than zero. Preferably, the reference value may be a value of the same type as values of the data set to be calibrated and may have the same unit or dimension as that of the data set to be calibrated.

When a normalization coefficient is provided using the reference value and a signal value at a cycle of the data set corresponding to the reference cycle, the normalization coefficient may be provided as a ratio of the signal value at the cycle corresponding to the reference cycle of the data set to the reference value.

According to a more specific embodiment of the present invention, the method further comprises, between steps (a-1) and (a-2), the following steps:
(a-1-1) applying a threshold value to the first data set; and
(a-1-2) proceeding to the next step (a-2) when the first data set has a signal value exceeding the threshold value.

This embodiment represents a process for filtering the first data set.

In general, in the case of a negative sample that does not contain a target nucleic acid sequence, not only the first data set obtained in step (a-1) but also the second data set obtained in step (a-2) will have significantly low signal values at all cycle numbers. In particular, the second data set from a negative sample will not exhibit a pattern such as an amplification curve or a growth curve, that is, a slope value does not increase as the cycle number increases. This may make it difficult to obtain an S-shaped function that approximates a selected portion of the second data set. Therefore, it is preferable that the method of the present invention is not applied to a negative sample.

In this regard, the method of the present invention includes a step of filtering data sets, that is, removing data sets expected to be derived from negative samples and obtaining only data sets expected to be derived from positive samples.

In step (a-1-1), a threshold value is applied to the first data set of step (a-1) to distinguish a data set from a positive sample from a data set from a negative sample. Such distinction may be easily achieved by applying the threshold value. After application of the threshold value to the first data set of step (a-1), a data set having a signal value exceeding the threshold value is determined to be a data set derived from a positive sample, and a data set having all signal values below the threshold value is determined to be a data set derived from a negative sample.

Thereafter, in step (a-1-2), when the first data set has a signal value exceeding the threshold value, the next step (a-2) is proceeded to. Since a data set having a signal value exceeding the threshold value is expected to be derived from a positive sample, such a data set is expected to generate an S-shaped function that is best fitted according to the method of the present invention.

On the other hand, when the data set has all signal values below the threshold value, the next step (a-2) is not proceeded to. Since a data set having all signal values below the threshold value is expected to be derived from a negative sample, such a data set may fail to generate an S-shaped function that is best fitted according to the method of the present invention. A data set having all signal values below the threshold value may indicate absence of a target analyte without proceeding to step (a-2).

### (a-2) Obtaining a second data set

Next, according to the present embodiment, in step (a-2), a slope value at each cycle number for the first data set is calculated to obtain a second data set. The second data set includes a plurality of data points each having a cycle number and a slope value at the cycle number.

The second data set may be interchangeably used with a "slope data set."

As used herein, the term "slope value" refers to a change in a signal value at each data point, and may be interchangeably used with a "change value." Since a slope value or a change value may include a rate of change, the slope data set may include a rate-of-change data set.

According to one embodiment of the present invention, the slope value of step (a-2) is obtained by differentiation, difference, divided difference, ratio, or linear regression analysis of signal values.

Calculation of a slope value by differentiation generally includes calculating a function that approximates the first data set (*e.g.,* a raw data set), calculating a derivative from the approximated function, and obtaining a slope value at each cycle number using the derivative.

Calculation of a slope value by difference generally includes calculating a difference between a signal value at a cycle number for which a slope value is to be calculated (a target signal value) and a signal value at another cycle number (a reference signal value), and taking the calculated difference as a slope value at the target cycle number. The reference signal value may be, for example, a signal value at a cycle number immediately preceding or following the target cycle number, or a signal value at two or more cycle numbers before or after the target cycle number. Alternatively, the reference signal value may be an average of signal values at several cycle numbers before or after the target cycle number. Typically, the reference signal value may be a signal value at a cycle number immediately preceding the target cycle number.

Calculation of a slope value of a signal value by divided difference generally includes calculating a difference between a signal value at a cycle number for which a slope value is to be calculated (a target cycle number) and a signal value at another cycle number (a reference signal value), dividing the calculated difference by a difference between the target cycle number and the reference cycle number, and taking the result as a slope value at the target cycle number. The reference signal value may be, for example, a signal value at a cycle number immediately preceding or following the target cycle number, or a signal value at two or more cycle numbers before or after the target cycle number. A slope value determined by divided difference corresponds to an amount of change in a signal value per cycle number.

Calculation of a slope value by ratio generally includes calculating a ratio between two signal values at two cycle numbers. In one embodiment, a ratio at a target cycle number is calculated by dividing a signal value at the target cycle by a signal value at an immediately preceding cycle.

Calculation of a slope value by linear regression analysis generally includes obtaining a linear function that fits several data points including a data point for which a slope value is to be calculated (a target data point), and determining an amount of increase in a signal value per cycle number from the fitted function. The number of data points used to obtain the linear function may be two or more data points, and may be, for example, 2, 3, 4, 5, 6, 7, 8, 9, 10, 11, 12, 13, 14, or 15 or less.

Examples of linear regression analysis include, but are not limited to, a least squares method.

The least squares method may be represented by Equation 6 below. $\begin{matrix}m = \frac{{\sum }_{i = I - o}^{i + b} \left({x}_{i}-\overline{x}\right) \left({y}_{i}-\overline{y}\right)}{{\sum }_{i = I - a}^{i + b} {\left({x}_{i}-\overline{x}\right)}^{2}} \\ \overline{x} = \frac{{\sum }_{i = i - a}^{i + b} {x}_{i}}{n} , \overline{y} = \frac{{\sum }_{i = i - a}^{i + b} {H}_{i}}{n}\end{matrix}$ Equation 6

In the above equation, I is a cycle of a data point for which a slope is to be calculated, m is a slope of a data point at the I-th cycle, xᵢ is a cycle of an i-th cycle, yᵢ is a signal value measured at the i-th cycle, and Sᵢ is a slope value at the i-th cycle.

The "n" or "a+b+1" represents a number of data points used to calculate a slope at the I-th cycle and is referred to as a Linear Squares Method Range (LSMR). "a" is a value for calculating a minimum cycle among a set of data points used to calculate a slope at the I-th cycle, and "b" is a value for calculating a maximum cycle. "a" and "b" independently represent integers from 0 to 10, preferably from 1 to 5, more preferably from 1 to 3. Although it is advantageous for "a" and "b" to have the same value, they may differ from each other depending on a measurement target, a measurement environment, and a cycle at which a slope is to be measured.

According to one embodiment, calculation of a slope value at specific cycle number(s) may differ from calculation of a slope value at other cycle numbers. For example, when a slope value is obtained by a difference method, a slope value at a first cycle number or at a last cycle number may be obtained by a method different from the difference method; and when a slope value is obtained by linear regression analysis, a slope value at an initial cycle number (for example, a first cycle number and a second cycle number) or at a late cycle number (for example, a last cycle number and a cycle number immediately preceding the last cycle number) may be obtained by a method different from the linear regression analysis.

For example, a slope value at a first cycle number or at a late cycle number may be determined as zero. Alternatively, a slope value at a first cycle number may be determined as a signal value at the first cycle number or the second cycle number. Alternatively, a slope value at a first cycle number or at a late cycle number may be determined as a predetermined value.

According to one embodiment, the method may further include, between steps (a-1) and (a-3), a step of correcting slope values of one or more data points in the slope data set.

Examples of such correction include correcting abnormal signals in the slope data set, which may include detecting a cycle number having an abnormal signal value in the slope data set and correcting a slope value corresponding to the detected abnormal cycle number.

According to a more specific embodiment of the present invention, the method further comprises, between steps (a-2) and (a-3), the following steps:
(a-2-1) applying a threshold value to the second data set; and
(a-2-2) proceeding to the next step (a-3) when the second data set has a slope value exceeding the threshold value.

This embodiment represents a process for filtering the second data set.

This embodiment is identical to the process for filtering the first data set except that a threshold value is applied to the second data set instead of the first data set, and thus common contents therebetween are omitted in order to avoid excessive complexity of the present specification due to repetitive descriptions.

However, a threshold value applied in the process for filtering the second data set may be different from a threshold value applied in the process for filtering the first data set.

### (a-3) Calculating an S-shaped function that approximates a selected portion of the second data set

Next, according to the present embodiment, an S-shaped function that approximates a selected portion of the second data set is calculated.

According to one embodiment, step (a-3) may include the following sub-steps:
(a-3-1) selecting a portion of the second data set that exhibits a pattern of an amplification curve or a growth curve and is capable of being fitted to an S-shaped function; and (a-3-2) calculating an S-shaped function that approximates the selected portion.

In one embodiment, the selected portion in the second data set (the slope data set) of step (a-3) is a cycle range that exhibits a pattern of an amplification curve or a growth curve in the presence of a target analyte. That is, the selected portion is within a cycle range representing a shape of an amplification curve or a growth curve when the target analyte is present.

In general, when a target analyte is present, a slope data set such as the second data set exhibits a "bell-shaped curve," which is also known as a normal distribution. With respect to an axis of symmetry (a central axis), a left side of the bell-shaped curve exhibits an increasing pattern like an amplification curve or a growth curve, that is, a pattern in which slope values increase as the cycle number increases, whereas a right side of the bell-shaped curve exhibits a pattern in which slope values decrease as the cycle number increases.

Start and end cycles defining limits and bounds of the selected portion within the second data set of step (a-3) are as follows:

### (i) Start cycle

The start cycle of the selected portion may be selected in consideration of goodness of fit of an S-shaped function that approximates or fits the selected portion.

In one embodiment, the start cycle of the selected portion may be any one of cycle numbers from 1 to 30; any one of cycle numbers from 1 to 25; any one of cycle numbers from 1 to 20; any one of cycle numbers from 1 to 15; any one of cycle numbers from 1 to 10; or any one of cycle numbers from 1 to 5.

In a specific embodiment, the start cycle of the selected portion is any one of cycle numbers from 1 to 10 in the second data set.

### (ii) End cycle

The end cycle of the selected portion may be selected in consideration of goodness of fit of an S-shaped function that approximates or fits the selected portion.

In one embodiment, the end cycle of the selected portion may be any one of a last cycle number of the second data set or any one of five consecutive cycle numbers immediately preceding the last cycle number; any one of a last cycle number of the second data set or any one of four consecutive cycle numbers immediately preceding the last cycle number; any one of a last cycle number of the second data set or any one of three consecutive cycle numbers immediately preceding the last cycle number; any one of a last cycle number of the second data set or any one of two consecutive cycle numbers immediately preceding the last cycle number; or a last cycle number of the second data set or a cycle number immediately preceding the last cycle number.

In one embodiment, the end cycle of the selected portion is a last cycle number in the second data set. For example, when a last cycle number in the slope data set is a 50th cycle number, the end cycle of the selected portion is the 50th cycle number in the second data set.

According to a more specific embodiment of the present invention, the selected portion of the second data set in step (a-3) is within a range from a first cycle number to a last cycle number in the second data set. In other words, each of the start cycle and the end cycle is selected within a range from the first cycle number to the last cycle number in the second data set.

The selected portion of the second data set may be selected directly by a user or automatically by a computer system, in consideration of the above-described requirements.

The selected portion of the second data set may include additional data points that were not originally present in the slope data set.

Thereafter, an S-shaped function that approximates the selected portion is calculated.

As used herein, the phrase "calculating an S-shaped function" refers to a process of deriving an S-shaped function that best fits data points of the selected portion. The process of calculating the S-shaped function may be referred to herein as S-shaped regression analysis. The S-shaped function may be calculated using fitting methods known in the art.

According to one embodiment, calculating the S-shaped function in step (a-3) includes fitting data points of the selected portion to a conventional S-shaped function known in the art, and determining a best-fitting function and parameters thereof.

According to another embodiment, calculating the S-shaped function in step (a-3) includes determining parameters of a specific S-shaped function. Specifically, calculating an S-shaped regression function in step (a-3) includes fitting data points of the selected portion to a specific S-shaped function, and determining parameters of the best-fitting function.

According to one embodiment, a computer system is used to calculate a function fitted to data points of the selected portion. The computer system includes a computer-readable storage medium having stored therein a computer program capable of performing regression analysis, and a processor capable of executing the computer program.

As described above, the S-shaped function may be a predetermined function, and the regression analysis may be a process of determining parameters of the S-shaped function.

Specifically, when a cycle at a data point is set as an independent variable x and a signal value at the cycle is set as a dependent variable y, the dependent variable may be expressed as a polynomial function of the independent variable.

### Step (b): Obtaining an n-th order differential function and an n+1-th order differential function from the S-shaped function (120)

Next, the method of the present invention comprises step (b) of obtaining an nth order differential function and an n+1th order differential function from the S-shaped function, wherein n is a natural number.

This step includes differentiating the S-shaped function to calculate a first-order, second-order, n-th order, or n+1-th order differential function (derivative).

As can be confirmed from Example 3 described below, n is a natural number.

According to one embodiment of the present invention, n is a natural number selected from 1 to 10. More specifically, n is a natural number selected from 1 to 7, more specifically selected from 1 to 5, and even more specifically selected from 1 to 4.

### Step (c): Obtaining a first-order function connecting i) a first extreme point of the n-th order differential function or the n+1-th order differential function and ii) a second extreme point of the n+1-th order differential function (130)

Subsequently, the method of the present invention comprises step (c) of obtaining a first order function connecting i) a first extreme point of the nth order differential function or the n+1th order differential function and ii) a second extreme point of the n+1th order differential function.

As used herein, the term "extreme point" refers to a point at which a function changes from increasing to decreasing or from decreasing to increasing, and is also referred to as a local or relative maximum point or a local or relative minimum point.

As used herein, the term "global maximum point" refers to a point at which a function value is the maximum in the function, and the term "global minimum point" refers to a point at which a function value is the minimum in the function.

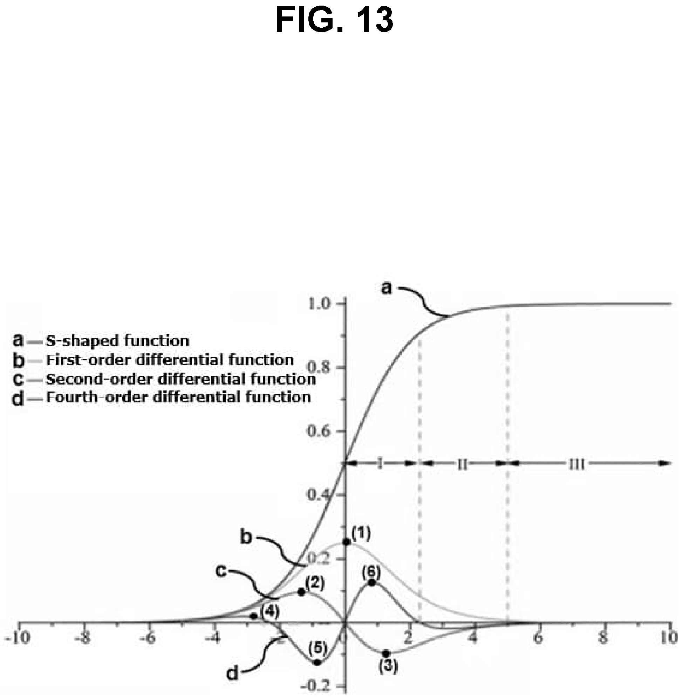

FIG. 13 illustrates an S-shaped function (a), and first-order (b), second-order (c), and fourth-order (d) differential functions thereof.

In the first-order differential function of FIG. 13, point (1) is an extreme point (specifically, a local maximum point) and also a global maximum point. In the second-order differential function, point (2) is an extreme point (specifically, a local maximum point) and also a global maximum point, and point (3) is an extreme point (specifically, a local minimum point) and also a global minimum point. In the fourth-order differential function, point (4) is an extreme point (specifically, a local maximum point) but is not a global maximum point, point (5) is an extreme point (specifically, a local minimum point) and also a global minimum point, and point (6) is an extreme point (specifically, a local maximum point) and also a global maximum point.

As can be seen in FIG. 13, an extreme point and a global maximum point (or a global minimum point) may be the same or may be different.

In this regard, it is necessary to clearly distinguish between an extreme point and a global maximum point (or a global minimum point) in detecting a target analyte in a sample.

One of the features of the present invention is that a first-order function connecting a second extreme point of an n+1-th order differential function and a first extreme point of an n-th order differential function or an n+1-th order differential function is used. Accordingly, according to the present invention, a first-order function connecting a second extreme point (a minimum point) and a third extreme point (a maximum point) of the fourth-order differential function shown in FIG. 13 is not used.

### Step (d): Detecting a target analyte in a sample using an intersection point between the S-shaped function and the first-order function (140)

Finally, the method of the present invention comprises step (d) of detecting the target analyte in the sample by using the intersection point of the S-shaped function of step (a) with the first order function of step (c).

The most significant feature of the present invention in detecting a target analyte in a sample is that, instead of simply using a maximum point or a minimum point of an n-th order differential function of an S-shaped function, an intersection point between the S-shaped function and a first-order function connecting a first extreme point of an n-th order differential function or an n+1-th order differential function for the S-shaped function and a second extreme point of the n+1-th order differential function is used. As confirmed in examples described below, this approach provides suitability for quantification and excellent quantitative accuracy and precision, even in the presence of inter-experiment variations in real-time PCR reactions.

As used herein, the terms "detect," "detecting," or "detection" include both qualitative and quantitative determination of a target analyte. As used herein, the term "qualitative" or "qualitative analysis" refers to determining presence of a target analyte in a sample using the method of the present invention, whereas the term "quantitative" or "quantitative analysis" refers to determining an initial amount of a target analyte in a sample using the method of the present invention.

According to one embodiment of the present invention, using the intersection point in step (d) includes determining the intersection point as a quantification cycle (Cq) value. The Cq value is used for qualitative or quantitative analysis of a target analyte in a sample.

Although Cq is used substantially interchangeably with Ct, in the present specification, Ct determined using a threshold and Cq determined according to the method of the present invention are distinguished from each other.

According to a more specific embodiment of the present invention, determining the intersection point as a quantification cycle (Cq) value includes determining a cycle number of the intersection point as the Cq value.

The intersection point, more specifically the determined Cq (Quantification Cycle) value, may be used for qualitative or quantitative analysis of a target analyte in a sample as follows.

### a. Qualitative analysis

In qualitative analysis, a sample that does not have the intersection point (specifically, a determined Cq value) or that has an intersection point (specifically, a determined Cq value) greater than a last cycle in a data set of step (a-1) or step (a-2) is determined to not contain a target analyte (*i.e.,* a negative sample); whereas a sample that has an intersection point (specifically, a determined Cq value) less than or equal to a last cycle in a data set of step (a-1) or step (a-2) is determined to contain a target analyte (*i.e.,* a positive sample).

For example, when an intersection point (specifically, a determined Cq value) obtained from an unknown sample according to the method of the present invention is 35 and a last cycle in a data set of step (a-1) or step (a-2) is 50, the sample is determined to be positive; whereas when an intersection point (specifically, a determined Cq value) is 70 and a last cycle in a data set of step (a-1) or step (a-2) is 50, the sample is determined to be negative.

### b. Quantitative analysis

In quantitative analysis, a dilution series of a standard target analyte is applied to amplification reactions to obtain a plurality of amplification curves, and intersection points (specifically, Cq values) are then determined from the amplification curves according to the method of the present invention described above. Thereafter, the intersection points (specifically, Cq values) are plotted against log-transformed concentrations of the standard analyte to obtain a standard curve for quantification. Subsequently, an unknown sample (a sample having an unknown initial amount) is applied to an amplification reaction to obtain an intersection point (specifically, a Cq value), and the initial amount of the unknown sample is then determined by applying the Cq value to the standard curve.

Qualitative or quantitative analysis using such intersection points (specifically, Cq values) may be performed according to various methods known in the art.

### Recording Medium, Apparatus, and Program

In another aspect of the present invention, there is provided a computer readable storage medium containing instructions to configure a processor to perform a method for detecting a target analyte in a sample, the method comprising:
(a) obtaining an S-shaped function representing a growth curve by an amplification reaction for a target analyte;
(b) obtaining an n-th order differential function and an n+1-th order differential function from the S-shaped function, wherein n is a natural number;
(c) obtaining a first-order function connecting (i) a first extreme point of the n-th order differential function or the n+1-th order differential function and (ii) a second extreme point of the n+1-th order differential function; and
(d) detecting the target analyte in the sample by using an intersection point between the S-shaped function of step (a) and the first-order function of step (c).

In still another aspect of the present invention, there is provided a computer program to be stored on a computer readable storage medium, to configure a processor to perform a method for detecting a target analyte in a sample, the method comprising:
(a) obtaining an S-shaped function representing a growth curve by an amplification reaction for a target analyte;
(b) obtaining an n-th order differential function and an n+1-th order differential function from the S-shaped function, wherein n is a natural number;
(c) obtaining a first-order function connecting (i) a first extreme point of the n-th order differential function or the n+1-th order differential function and (ii) a second extreme point of the n+1-th order differential function; and
(d) detecting the target analyte in the sample by using an intersection point between the S-shaped function of step (a) and the first-order function of step (c).

In another aspect of the present invention, there is provided an apparatus for detecting a target analyte in a sample comprising (a) a computer processor; and (b) the computer readable storage medium of the present invention coupled to the computer processor.

According to one embodiment of the present invention, the apparatus of the present invention may further include a reaction vessel capable of accommodating a sample and a signal-generating means, a temperature control means for controlling a temperature of the reaction vessel, and/or a detector for detecting a signal during an amplification cycle.

The recording medium, apparatus, and computer program of the present invention are configured to enable execution of the above-described method of the present invention on a computer. Therefore, the common descriptions among them are omitted in order to avoid undue redundancy leading to the complexity of this specification.

When executed by a processor, program instructions cause the processor to perform the above-described method of the present invention. Program instructions for performing a method for detecting a target analyte in a sample may include the following instructions: (i) an instruction to obtain an S-shaped function representing a growth curve by an amplification reaction for a target analyte; (ii) an instruction to obtain an n-th order differential function and an n+1-th order differential function from the S-shaped function; (iii) an instruction to obtain a first-order function connecting (i) a first extreme point of the n-th order differential function or the n+1-th order differential function and (ii) a second extreme point of the n+1-th order differential function; and (iv) an instruction to detect a target analyte in the sample by using the intersection point between the S-shaped function and the first-order function (e.g., displaying the result on an output device).

The method of the present invention is executed by a processor, and the processor may be a processor in a stand-alone computer, a network-attached computer, or a processor in a data acquisition device such as a real-time PCR apparatus.

According to one embodiment of the present invention, the computer-readable storage medium is a non-transitory computer-readable storage medium.

The computer-readable storage medium includes various storage media known in the art, including, but not limited to, CD-R, CD-ROM, DVD, flash memory, floppy disk, hard drive, portable HDD, USB, magnetic tape, MiniDisc, non-volatile memory card, EEPROM, optical disk, optical storage medium, RAM, ROM, system memory, and web servers.

The process and results of detecting a target analyte in a sample may be provided in various manners. For example, the process and results of detecting the target analyte in the sample may be provided to a separate system such as a desktop computer system via a network connection (*e.g.,* LAN, VPN, internet, or intranet) or via a direct connection (*e.g.,* USB or other direct wired connection or wireless connection), or may be provided on a portable medium such as a CD, DVD, floppy disk, or portable HDD. Similarly, the process and results of detecting the target analyte in the sample may be provided from a client such as a laptop or desktop computer system to a server system via a network connection (*e.g.,* LAN, VPN, internet, intranet, or a wireless communication network).

Instructions for configuring a processor to perform the present invention may be included in a logic system. Such instructions may be provided in a software storage medium (*e.g.,* a portable HDD, USB, floppy disk, CD, or DVD), and may also be downloadable and stored in a memory module (*e.g.,* a hard drive or other memory such as local or attached RAM or ROM). Computer code for performing the present invention may be executed in various coding languages, such as C, C++, Java, Visual Basic, VBScript, JavaScript, Perl, and XML. In addition, various languages and protocols may be used for external and internal storage and transmission of signals and commands according to the present invention.

A computer processor may be configured such that a single processor performs all of the above-described functions. Alternatively, a processor unit may be configured such that a plurality of processors perform respective functions.

According to one embodiment of the present invention, the processor may be implemented by installing software in a conventional apparatus for detecting a target nucleic acid sequence (*e.g.,* a real-time PCR apparatus).

### II. Method for determining a quantification cycle (Cq) value for a target analyte in a sample (second aspect)

In another aspect of the present invention, there is provided a method for determining a quantification cycle (Cq) value for a target analyte in a sample, comprising:
(a) obtaining an S-shaped function representing a growth curve by an amplification reaction for a target analyte;
(b) obtaining an n-th order differential function and an n+1-th order differential function from the S-shaped function, wherein n is a natural number;
(c) obtaining a first-order function connecting (i) a first extreme point of the n-th order differential function or the n+1-th order differential function and (ii) a second extreme point of the n+1-th order differential function; and
(d) determining an intersection point between the S-shaped function of step (a) and the first-order function of step (c) as a Cq value.

Since the present invention relates to a method for determining a Cq value using an intersection point between the S-shaped function of step (a) and the first-order function of step (c) according to the method of the first aspect of the present invention described above, the common descriptions among them are omitted in order to avoid undue redundancy leading to the complexity of this specification.

According to one embodiment, the method is a computer-implemented method.

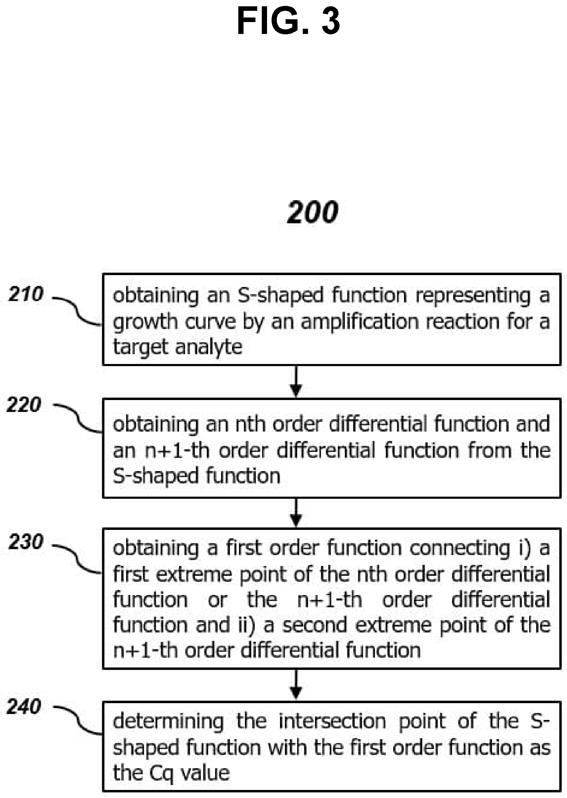

FIG. 3 is a flowchart illustrating processes for performing the method of the present invention according to one embodiment. The method of the present invention will be described below with reference to FIG. 3.

### Step (a): Obtaining an S-shaped function representing a growth curve (210)

First, the method of the present invention comprises step (a) of obtaining an S-shaped function representing a growth curve by an amplification reaction for a target analyte.

According to one embodiment of the present invention, the target analyte is a target nucleic acid molecule.

According to one embodiment, the S-shaped function is selected from the group consisting of a sigmoid function, a logistic function, a Gompertz function, a Chapman function, and a Richard function.

According to one embodiment of the present invention, the step of obtaining the S-shaped function of step (a) comprises the following steps:
(a-1) obtaining a first data set representing a growth curve by an amplification reaction for a target analyte, wherein the first data set includes a plurality of data points, each having a cycle number and a signal value at the cycle number;
(a-2) calculating a slope value at each cycle number for the first data set to obtain a second data set, wherein the second data set includes a plurality of data points, each having a cycle number and a slope value at the cycle number; and
(a-3) calculating an S-shaped function that approximates a selected portion of the second data set.

According to a more specific embodiment, the first data set of step (a-1) is a raw data set, a mathematically modified data set of the raw data set, a normalized data set of the raw data set, or a normalized data set of the mathematically modified data set.

According to a more specific embodiment of the present invention, the method further comprises, between steps (a-1) and (a-2), the following steps:
(a-1-1) applying a threshold value to the first data set; and
(a-1-2) proceeding to the next step (a-2) when the first data set has a signal value exceeding the threshold value.

According to a more specific embodiment, the slope value of step (a-2) is obtained by differentiation, difference, divided difference, ratio, or linear regression analysis of signal values.

According to a more specific embodiment of the present invention, the method further comprises, between steps (a-2) and (a-3), the following steps:
(a-2-1) applying a threshold value to the second data set; and
(a-2-2) proceeding to the next step (a-3) when the second data set has a slope value exceeding the threshold value.

According to one embodiment, step (a-3) may include the following sub-steps:
(a-3-1) selecting a portion of the second data set that exhibits a pattern of an amplification curve or a growth curve and is capable of being fitted to an S-shaped function; and
(a-3-2) calculating an S-shaped function that approximates the selected portion.

According to a more specific embodiment of the present invention, the selected portion of the second data set in step (a-3) is within a range from a first cycle number to a last cycle number in the second data set.

According to one embodiment, calculating the S-shaped function in step (a-3) includes fitting data points of the selected portion to a conventional S-shaped function known in the art, and determining a best-fitting function and parameters thereof.

According to another embodiment, calculating the S-shaped function in step (a-3) includes determining parameters of a specific S-shaped function. Specifically, calculating an S-shaped regression function in step (a-3) includes fitting data points of the selected portion to a specific S-shaped function, and determining parameters of the best-fitting function.

### Step (b): Obtaining an n-th order differential function and an n+1-th order differential function from the S-shaped function (220)

Next, the method of the present invention comprises step (b) of obtaining an nth order differential function and an n+1th order differential function from the S-shaped function, wherein n is a natural number.

According to one embodiment of the present invention, n is a natural number selected from 1 to 10.

### Step (c): Obtaining a first-order function connecting (i) a first extreme point of the n-th order differential function or the n+1-th order differential function and (ii) a second extreme point of the n+1-th order differential function (230)

Subsequently, the method of the present invention comprises step (c) of obtaining a first order function connecting (i) a first extreme point of the nth order differential function or the n+1th order differential function and (ii) a second extreme point of the n+1th order differential function.

### Step (d): Determining an intersection point between the S-shaped function and the first-order function as a Cq value (240)

Finally, the method of the present invention comprises step (d) of determining the intersection point of the S-shaped function of step (a) with the first order function of step (c) as a Cq value.

### Recording Medium, Apparatus, and Program

In another aspect of the present invention, there is provided a computer readable storage medium containing instructions to configure a processor to perform a method for determining a quantification cycle (Cq) value for a target analyte in a sample, the method comprising:
(a) obtaining an S-shaped function representing a growth curve by an amplification reaction for a target analyte;
(b) obtaining an n-th order differential function and an n+1-th order differential function from the S-shaped function, wherein n is a natural number;
(c) obtaining a first-order function connecting (i) a first extreme point of the n-th order differential function or the n+1-th order differential function and (ii) a second extreme point of the n+1-th order differential function; and
(d) determining an intersection point between the S-shaped function of step (a) and the first-order function of step (c) as a Cq value.

In still another aspect of the present invention, there is provided a computer program to be stored on a computer readable storage medium, to configure a processor to perform a method for determining a quantification cycle (Cq) value for a target analyte in a sample, the method comprising:
(a) obtaining an S-shaped function representing a growth curve by an amplification reaction for a target analyte;
(b) obtaining an n-th order differential function and an n+1-th order differential function from the S-shaped function, wherein n is a natural number;
(c) obtaining a first-order function connecting (i) a first extreme point of the n-th order differential function or the n+1-th order differential function and (ii) a second extreme point of the n+1-th order differential function; and
(d) determining an intersection point between the S-shaped function of step (a) and the first-order function of step (c) as a Cq value.

In another aspect of the present invention, there is provided an apparatus for determining a quantification cycle (Cq) value for a target analyte in a sample, comprising (a) a computer processor; and (b) the computer readable storage medium of the present invention coupled to the computer processor.

When executed by a processor, program instructions cause the processor to perform the above-described method of the present invention. Program instructions for performing a method for determining a quantification cycle (Cq) value for a target analyte in a sample may include the following instructions: (i) an instruction to obtain an S-shaped function representing a growth curve by an amplification reaction for a target analyte; (ii) an instruction to obtain an n-th order differential function and an n+1-th order differential function from the S-shaped function; (iii) an instruction to obtain a first-order function connecting (i) a first extreme point of the n-th order differential function or the n+1-th order differential function and (ii) a second extreme point of the n+1-th order differential function; and (iv) an instruction to determine an intersection point between the S-shaped function and the first-order function as a Cq value (*e.g.,* displaying the Cq value on an output device).

According to one embodiment, the computer readable storage medium is a non-transitory computer-readable storage medium.

### EFFECTS OF THE INVENTION

The features and advantages of the present invention are summarized as follows:
(a) The present invention utilizes, for detection of a target analyte, an intersection point between (i) a function representing an amplification curve for the target analyte and (ii) a first-order function connecting a first extreme point of an n-th order differential function or an n+1-th order differential function of the function representing the amplification curve for the target analyte and a second extreme point of the n+1-th order differential function.
(b) The present invention provides superior quantitative accuracy and precision compared to conventional methods, even in the presence of inter-experiment variations in real-time PCR reactions.
(c) The present invention exhibits excellent quantitative accuracy and is therefore applicable to complex samples in which experimental variations may occur, and may be usefully applied in molecular biology research where accurate measurement of DNA amounts is required.

### BRIEF DESCRIPTION OF THE DRAWINGS

FIG. 1 is a flowchart illustrating processes for performing the method of the present invention according to one embodiment of the present invention.

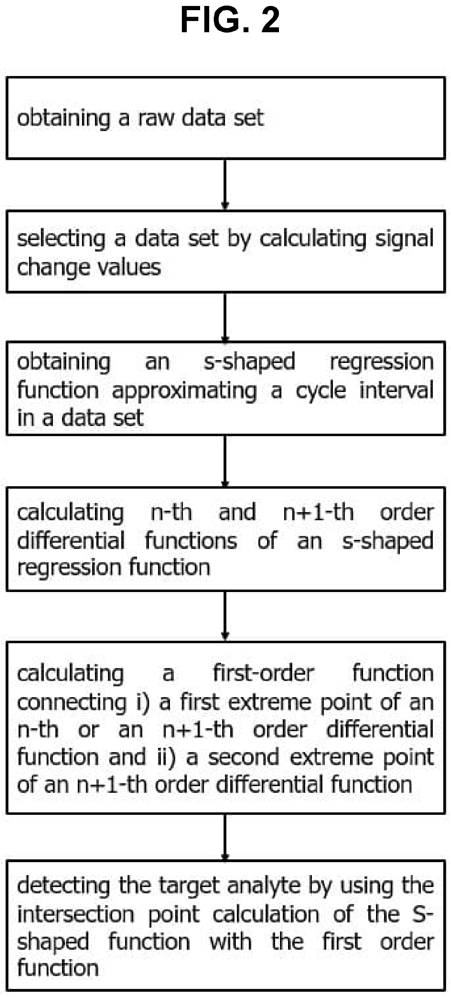

FIG. 2 is a flowchart illustrating processes for performing the method of the present invention according to a more specific embodiment.
FIG. 3 is a flowchart illustrating processes for performing the method of the present invention according to another embodiment of the present invention.
FIG. 4 illustrates raw data sets obtained from samples containing target analytes at various concentrations and a sample not containing a target analyte according to one embodiment of the present invention (solid line: NG 100 pg; - ○-: NG 10 pg; -□-: NG 1 pg; -△-: NG 100 fg; and -X-: NTC).
FIG. 5 illustrates data sets of change values obtained from samples containing target analytes at various concentrations and a sample not containing the target analyte according to one embodiment of the present invention ((a): NG 100 pg; (b): NG 10 pg; (c): NG 1 pg; and (d): NG 100 fg).
FIG. 6 illustrates curves representing a Richard function obtained from samples containing *Neisseria gonorrhoeae* (NG) genomic DNA at various concentrations as a target analyte according to one embodiment of the present invention ((a): real-time PCR raw data of NG without baseline subtraction; (b): results of Richard function fitting; and (c): baseline-subtracted Richard function fitting data).

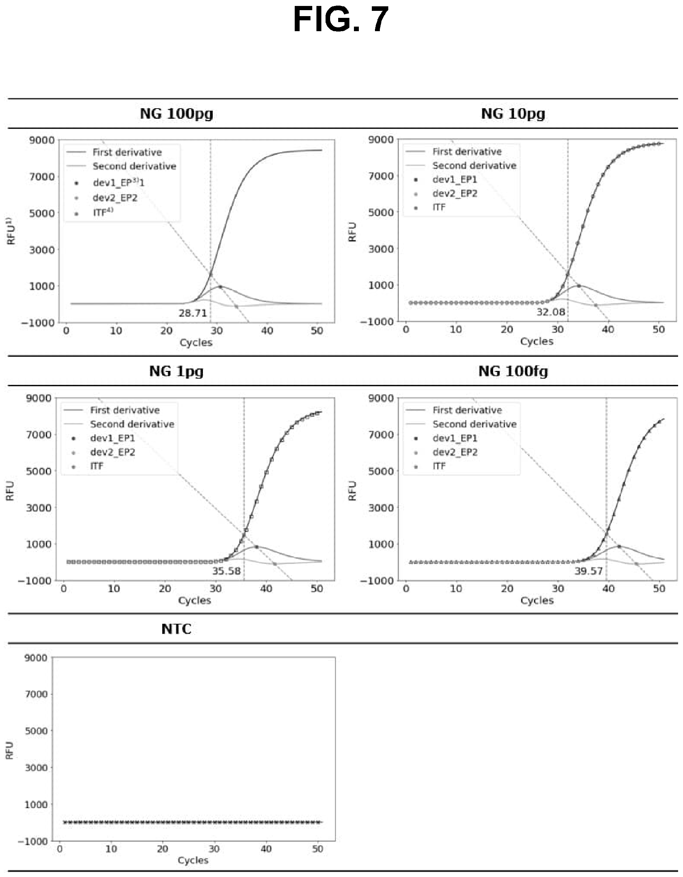

FIG. 7 illustrates a process of detecting a target analyte by determining, as a Cq (cycle quantification) value, a cycle number of an intersection point between a curve represented by a Richard function and a first-order function obtained according to one embodiment of the present invention. The first-order function is a linear function passing through a first extreme point of a first-order differential function and a second extreme point of a second-order differential function obtained by differentiating the curve represented by the Richard function. In FIG. 7, dev1 indicates a first-order differential function, EP1 indicates a first extreme point, EP2 indicates a second extreme point, and ITF indicates an intersection point between the two functions.
FIG. 8 illustrates a process of detecting a target analyte by determining, as a Cq (cycle quantification) value, a cycle number of an intersection point between a curve represented by a Richard function and a first-order function obtained according to one embodiment of the present invention. The first-order function is a linear function passing through a first extreme point and a second extreme point of a second-order differential function obtained by differentiating the curve represented by the Richard function. In FIG. 8, dev2 indicates a second-order differential function.
FIG. 9 illustrates results of determining Cq values using a linear function connecting a first extreme point of an n-th order differential function or an n+1-th order differential function and a second extreme point of an n+1-th order differential function and a curve represented by a Richard function, obtained from a raw data set derived from a sample containing NG 100 pg as a target analyte (where n is from 1 to 3).
FIG. 10 illustrates raw data sets obtained from samples containing *Neisseria gonorrhoeae* (NG) genomic DNA at various concentrations as a target analyte through six repeated experiments.
FIG. 11 illustrates a standard curve generated by using samples containing a target analyte at four different concentrations, determining a cycle number of an intersection point between an S-shaped regression function and a first-order function passing through a first extreme point of a first-order differential function and a second extreme point of a second-order differential function, and plotting the determined cycle numbers.
FIG. 12 illustrates a standard curve generated by using samples containing a target analyte at four different concentrations, determining a cycle number of an intersection point between an S-shaped regression function and a first-order function passing through a first extreme point and a second extreme point of a second-order differential function, and plotting the determined cycle numbers.
FIG. 13 illustrates an S-shaped function (a), and first-order (b), second-order (c), and fourth-order (d) differential functions thereof.

The present invention will now be described in further detail by examples. It would be obvious to those skilled in the art that these examples are intended to be more concretely illustrative and the scope of the present invention as set forth in the appended claims is not limited to or by the examples.

### Examples

### Example 1: Detection of a Target Analyte Using a First-Order Function Connecting a First Extreme Point of an n-th (n=1) Order Differential Function and a Second Extreme Point of an n+1-th (n=1) Order Differential Function of an S-Shaped Regression Function Obtained from a Raw Data Set

It was investigated whether a target analyte could be detected using a first-order function connecting a first extreme point of a first-order differential function and a second extreme point of a second-order differential function of an S-shaped regression function obtained from a raw data set.

### Example 1-1: Performance of Real-Time Polymerase Chain Reaction and Acquisition of a Raw Data Set

*Neisseria gonorrhoeae* (NG) genomic DNA (supplier: Coram Deo Lab Co., Ltd.; accession number: ATCC 700825) was used as a target analyte. In order to amplify the target analyte and generate a signal from amplification products, real-time polymerase chain reaction was performed using primers and a TaqMan probe listed in Table 1 below.

**Table 1**

| Name | Type | Sequence (5'→ 3') | Sequence ID No. |
|---|---|---|---|
| NG_F | Primer | TACGCCTGCTACTTTCACGCT | SEQ ID NO: 1 |
| NG_R | Primer | CAATGGATCGGTATCACTCGC | SEQ ID NO: 2 |
| NG_P | Probe | [FAM]TGCCCCTCATTGGCGTGTTTCG[BHQ-1] | SEQ ID NO: 3 |

In Table 1, BHQ represents a quencher (Black Hole Quencher).

The real-time polymerase chain reaction was performed by loading a 20 µl reaction mixture into a real-time PCR instrument (CFX96 Real-time Cycler, Bio-Rad), the reaction mixture comprising 10 pmole of a forward primer and a reverse primer (SEQ ID Nos: 1 and 2), 5 pmole of a TaqMan probe (SEQ ID No: 3), Neisseria gonorrhoeae genomic DNA as a target analyte (100 pg, 10 pg, 1 pg, or 100 fg), sterile distilled water (NTC) as a negative control, and 10 µl of a 2× Master Mix (final concentrations: 200 µM dNTPs, 2.5 mM MgCl₂, and 1.6 U Taq DNA polymerase) (Solgent, Korea). The reaction mixture was incubated at 95°C for 15 minutes, followed by 50 cycles of reactions consisting of 95°C for 30 seconds, 60°C for 60 seconds, and 72°C for 30 seconds. Fluorescence detection was performed at 60°C at each cycle.

Through the real-time polymerase chain reaction, a raw data set for the target analyte (raw dataset; without baseline subtraction) was obtained from the instrument.

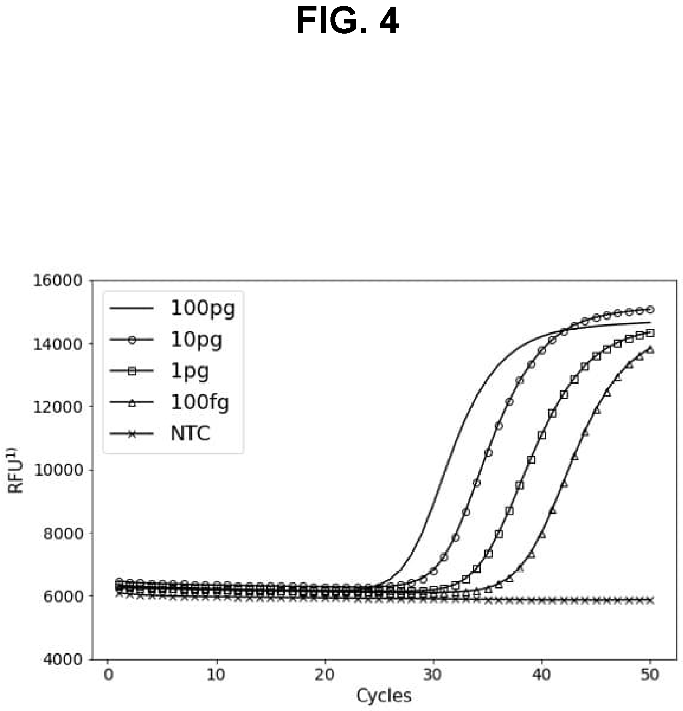

Raw data sets obtained from samples containing the target analyte at various concentrations and a sample not containing the target analyte are shown in FIG. 4 (solid line: NG 100 pg; -○-: NG 10 pg; -□-: NG 1 pg; -△-: NG 100 fg; -X-: NTC).

### Example 1-2: Acquisition of a Data Set of Change Values

From the raw data set obtained in Example 1-1, change values of signal values at each cycle number were calculated from signal values to obtain a data set of change values.

Calculation of change values from signal values was performed by a difference method as shown in Equation 7 below. $Δ {y}_{i} = {y}_{i} - {y}_{i - 1} \left(i=1 Δ{y}_{1}=0\right)$ Equation 7

In the above equation, i is a cycle number of a data set; yᵢ is a signal value at the i-th cycle number; and Δyᵢ is a change value of the signal value at the i-th cycle number.

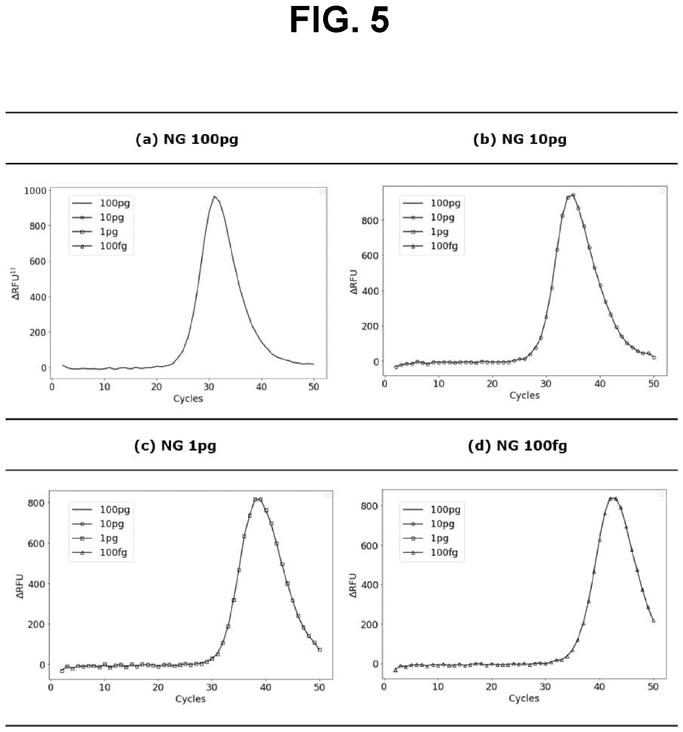

Data sets of change values obtained from samples containing the target analyte at various concentrations and a sample not containing the target analyte are shown in FIG. 5 ((a): NG 100 pg; (b): NG 10 pg; (c): NG 1 pg; and (d): NG 100 fg).

### Example 1-3: Selection of a Raw Data Set for Obtaining an S-Shaped Regression Function

Among the raw data sets obtained in Example 1-2, raw data sets for obtaining an S-shaped regression function were selected.

As a method for selecting raw data sets, a screening threshold was applied to each raw data set. Thereafter, a data set having values equal to or greater than the applied screening threshold was determined to be a raw data set for obtaining an S-shaped regression function and the next step was proceeded to, whereas a data set having only values below the applied screening threshold was determined to be negative without obtaining an S-shaped regression function.

In this example, a screening threshold of 100 (unit: ΔRFU (relative fluorescence unit)) was used.

As a result of the selection, data sets obtained from samples containing the target analyte at various concentrations included values equal to or greater than the screening threshold and were determined to be data sets for obtaining an S-shaped regression function, and therefore the following step was proceeded to. In contrast, data sets obtained from samples not containing the target analyte included only values below the screening threshold and were determined to be negative samples without proceeding to the following step.

### Example 1-4: Obtaining an S-Shaped Regression Function for a Selected Interval in a Raw Data Set

From the raw data sets determined to proceed to the following step (Example 1-4) in Example 1-3, a cycle interval for obtaining an S-shaped regression function was selected. The cycle interval was set as a range from a first cycle number to a last cycle number in the change-value data set.

Thereafter, an S-shaped regression function approximating the selected interval was determined. As the S-shaped regression function, the Richard function of Equation 5 below was used. $f \left(x\right) = \frac{{F}_{max}}{{\left[1+{e}^{\left(-\frac{1}{b}\left(x-c\right)\right)}\right]}^{d}} + {F}_{b}$ Equation 5

In the above equation, x is a cycle number; F(x) is reaction fluorescence at the cycle; Fₘₐₓ is maximum reaction fluorescence; b is a slope of the curve; c is a fractional cycle at which the reaction fluorescence is reached; d is a Richard coefficient; and F_{b} is background fluorescence.

Fitting of the selected interval in the raw data set to the Richard function was performed by a conventionally known LM algorithm (Levenberg-Marquardt algorithm; Christian Kanzow et al., JCAM, 172(2):375 (2004)). Using the LM algorithm, a Richard function approximating the selected interval was obtained.

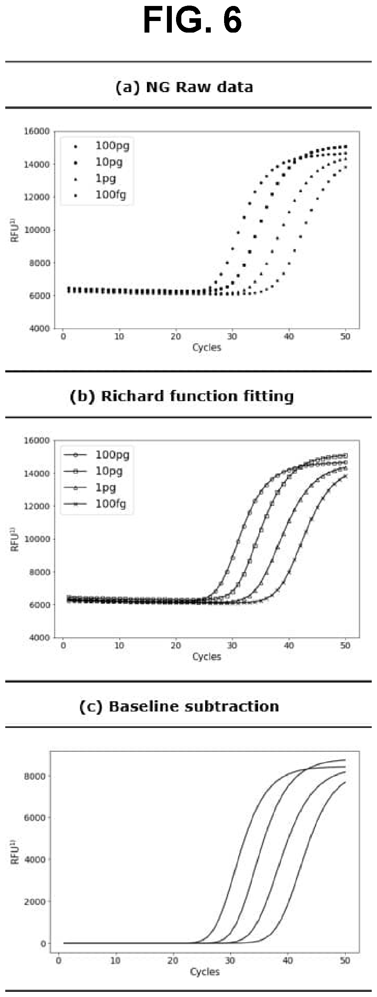

Curves representing Richard functions obtained from samples containing Neisseria gonorrhoeae (NG) genomic DNA at various concentrations as a target analyte are shown in FIG. 6, and values of parameters of each Richard function are shown in Table 2 below.

**Table 2**

| Parameter | Parameter Value | | | |
|---|---|---|---|---|
| | 100 pg | 10 pg | 1 pg | 100 fg |
| F_{b} | 6189 | 6319 | 6189 | 6149 |
| Fmax | 8421 | 8800 | 8410 | 8302 |
| b | 3.031 | 3.244 | 3.416 | 3.127 |
| c | 25.53 | 27.86 | 32.44 | 38.47 |
| d | 5.507 | 7.053 | 5.183 | 3.155 |

### Example 1-5: Detection of a Target Analyte Using an Intersection Point Between an S-Shaped Regression Function and a First-Order Function Connecting a First Extreme Point of a First-Order Differential Function of the S-Shaped Regression Function and a Second Extreme Point of a Second-Order Differential Function

Differentiation was applied to the curve represented by the Richard function obtained in Example 1-4 to calculate a first extreme point of a first-order differential function and a second extreme point of a second-order differential function, and a first-order function passing through the first extreme point of the first-order differential function and the second extreme point of the second-order differential function was calculated.

Thereafter, an intersection point between the curve represented by the Richard function and the first-order function was calculated.

As shown in FIG. 7, the target analyte was detected by determining a cycle number of an intersection point between the curve represented by the Richard function obtained in Example 1-4 and the first-order function as a quantification cycle for detection, Cq (Quantification Cycle).

Thereafter, a sample having a cycle number less than or equal to a last cycle number of the data set, which was the 50th cycle, was determined to be positive, and a sample having a cycle number greater than the 50th cycle was determined to be negative. The results are shown in Table 3 below.

As shown in Table 3, cycle numbers obtained from samples containing the target analyte at various concentrations were 28.71, 32.08, 35.58, and 39.57, respectively, and since all of the cycle numbers were less than 50, all of the samples were determined to be positive.

**Table 3**

| Target analyte | Positive | | | | Negative |
|---|---|---|---|---|---|
| Concentration | 100 pg | 10 pg | 1 pg | 100 fg | - |
| Screening threshold | 100 △RFU | | | | |
| Acquisition of S-shaped regression function | Proceeded | Proceeded | Proceeded | Proceeded | Not proceeded |
| Cycle number of intersection point of first-order function | 28.71 | 32.08 | 35.58 | 39.57 | - |
| Detection result of target analyte based on intersection point of first-order function | Positive | Positive | Positive | Positive | Negative |

### Example 2: Detection of a Target Analyte Using a First-Order Function Connecting a First Extreme Point and a Second Extreme Point of an (n+1)-th (n=1) Order Differential Function of an S-Shaped Regression Function Obtained from a Raw Data Set

Instead of determining a Cq value as a cycle number of an intersection point between a curve represented by a Richard function obtained from a raw data set and a first-order function passing through a first extreme point of a first-order differential function and a second extreme point of a second-order differential function by applying differentiation to a curve represented by a Richard function obtained from a raw data set as in Example 1, differentiation was applied to the curve represented by the Richard function obtained from the raw data set to calculate a first extreme point of a second-order differential function and a second extreme point of the second-order differential function, and a first-order function passing through the first extreme point and the second extreme point of the second-order differential function was calculated.

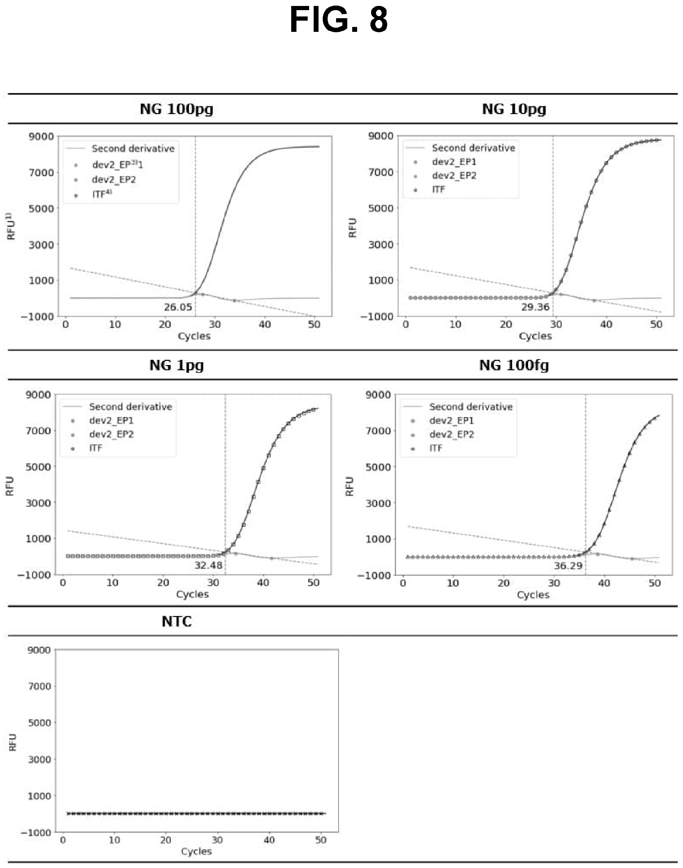

Thereafter, as shown in FIG. 8, a target analyte was detected by determining, as a Cq value, a cycle number of an intersection point between the curve represented by the Richard function obtained in Example 1-4 and the first-order function passing through the first extreme point and the second extreme point of the second-order differential function.

Thereafter, a sample having a cycle number less than or equal to a last cycle number of the data set, which was the 50th cycle, was determined to be positive, and a sample having a cycle number greater than the 50th cycle was determined to be negative. The results are shown in Table 4 below.

As shown in Table 4, cycle numbers obtained from samples containing the target analyte at various concentrations were 26.05, 29.36, 32.48, and 36.29, respectively, and since all of the cycle numbers were less than 50, all of the samples were determined to be positive.

**Table 4**

| Target analyte | Positive | | | | Negative |
|---|---|---|---|---|---|
| Concentration | 100 pg | 10 pg | 1 pg | 100 fg | - |
| Screening threshold | 100 △RFU | | | | |
| Acquisition of S-shaped regression function | Proceeded | Proceeded | Proceeded | Proceeded | Not proceeded |
| Cycle number of intersection point of first-order function | 26.05 | 29.36 | 32.48 | 36.29 | - |
| Detection result of target analyte based on intersection point of first-order function | Positive | Positive | Positive | Positive | Negative |

### Example 3: Determination of Cq Using a First-Order Function Connecting a First Extreme Point of an n-th Order Differential Function or an n+1-th Order Differential Function and a Second Extreme Point of an n+1-th Order Differential Function of an S-Shaped Regression Function Obtained from a Raw Data Set (n = 1 to 3)

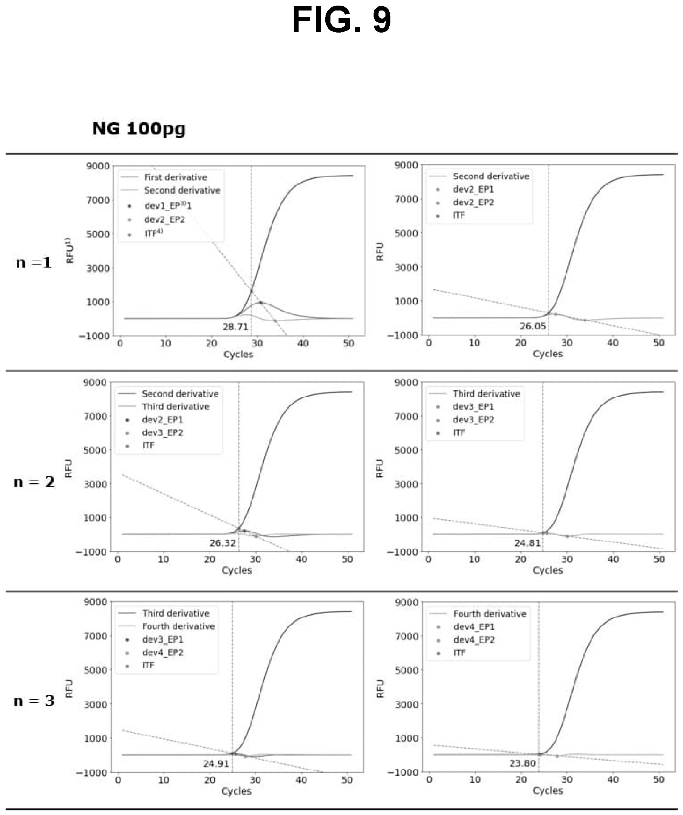

The experiment was performed in the same manner as in Example 1, except that a raw data set obtained from a sample containing NG 100 pg as a target analyte was used. From the curve represented by the Richard function obtained from the raw data set, cycle numbers of intersection points were determined as Cq values by using: 1) for n = 1, a first-order function passing through a first extreme point of a first-order differential function and a second extreme point of a second-order differential function, or a first-order function passing through a first extreme point of a second-order differential function and a second extreme point of the second-order differential function; 2) for n = 2, a first-order function passing through a first extreme point of a second-order differential function and a second extreme point of a third-order differential function, or a first-order function passing through a first extreme point of a third-order differential function and a second extreme point of the third-order differential function; and 3) for n = 3, a first-order function passing through a first extreme point of a third-order differential function and a second extreme point of a fourth-order differential function, or a first-order function passing through a first extreme point of a fourth-order differential function and a second extreme point of the fourth-order differential function. Cycle numbers of intersection points determined as Cq values using the above combinations are summarized in FIG. 9.

As can be seen in FIG. 9, it was confirmed that Cq values can be determined using extreme points up to a fourth-order differential function of the S-shaped regression function. In addition, since first-order to fourth-order differential functions of the S-shaped regression function all exhibited a pattern of increasing and then decreasing, it was confirmed that Cq values according to the present example could also be determined by using extreme points of fifth-order or higher-order differential functions.

### Comparative Example 1: Detection of a Target Analyte Using a Maximum Point of a First-Order Differential Function of an S-Shaped Regression Function Obtained from a Raw Data Set

Instead of determining a Cq value as a cycle number of an intersection point between a curve represented by a Richard function and a first-order function passing through a first extreme point of a first-order differential function and a second extreme point of a second-order differential function as in Example 1, a cycle number having a maximum value of a first-order differential function (First Derivative Maximum, FDM) was determined as a Cq value.

Thereafter, a sample having an FDM cycle number less than or equal to a last cycle number of the data set, which was the 50th cycle, was determined to be positive, and a sample having an FDM cycle number greater than the 50th cycle was determined to be negative. The results are shown in Table 5 below.

As shown in Table 5, FDM cycle numbers obtained from raw data sets of samples containing the target analyte at various concentrations were 30.70, 34.20, 38.06, and 42.06, respectively, and since all of the cycle numbers were less than 50, all of the samples were determined to be positive.

**Table 5**

| Target analyte | Positive | | | | Negative |
|---|---|---|---|---|---|
| Concentration | 100 pg | 10 pg | 1 pg | 100 fg | - |
| Screening threshold | 600 △RFU | | | | |
| Maximum signal value | 8421 | 8800 | 8410 | 8302 | 26 |
| Acquisition of S-shaped regression function | Proceeded | Proceeded | Proceeded | Proceeded | Not proceeded |
| FDM cycle number | 30.70 | 34.20 | 38.06 | 42.06 | - |
| Detection result of target analyte based on FDM | Positive | Positive | Positive | Positive | Negative |

### Comparative Example 2: Detection of a Target Analyte Using a Maximum Point of a Second-Order Differential Function of an S-Shaped Regression Function Obtained from a Raw Data Set

Instead of determining a Cq value as a cycle number of an intersection point between a curve represented by a Richard function and a first-order function passing through a first extreme point of a first-order differential function and a second extreme point of a second-order differential function as in Example 1, a cycle number having a maximum value of a second-order differential function (Second Derivative Maximum, SDM) was determined as a Cq value.

Thereafter, a sample having an SDM cycle number less than or equal to a last cycle number of the data set, which was the 50th cycle, was determined to be positive, and a sample having an SDM cycle number greater than the 50th cycle was determined to be negative. The results are shown in Table 6 below.

As shown in Table 6, SDM cycle numbers obtained from raw data sets of samples containing the target analyte at various concentrations were 27.55, 30.88, 34.50, and 38.65, respectively, and since all of the cycle numbers were less than 50, all of the samples were determined to be positive.

**Table 6**

| Target analyte | Positive | | | | Negative |
|---|---|---|---|---|---|
| Concentration | 100 pg | 10 pg | 1 pg | 100 fg | - |
| Screening threshold | 600 △RFU | | | | |
| Maximum signal value | 8421 | 8800 | 8410 | 8302 | 26 |
| Acquisition of S-shaped regression function | Proceeded | Proceeded | Proceeded | Proceeded | Not proceeded |
| SDM cycle number | 27.55 | 30.88 | 34.50 | 38.65 | - |
| Detection result of target analyte based on SDM | Positive | Positive | Positive | Positive | Negative |

### Test Example 1: Comparison of Measurement Values and Determination Results

Results of Examples 1 and 2 and results of Comparative Examples 1 and 2 according to conventional methods are summarized in Table 7 below.

**Table 7**

| Detection method | Target analyte | Positive | | | | Negative |
|---|---|---|---|---|---|---|
| | Concentration | 100 pg | 10 pg | 1 pg | 100 fg | - |
| Example 1 | Measured value | 28.71 | 32.08 | 35.58 | 39.57 | - |
| | Determination result | Positive | Positive | Positive | Positive | Negative |
| Example 2 | Measured value | 26.05 | 29.36 | 32.48 | 36.29 | - |
| | Determination result | Positive | Positive | Positive | Positive | Negative |
| Comparative Example 1 | Measured value | 30.70 | 34.20 | 38.06 | 42.06 | - |
| | Determination result | Positive | Positive | Positive | Positive | Negative |
| | Measured value | 27.55 | 30.88 | 34.50 | 38.65 | - |
| Comparative Example 2 | Determination result | Positive | Positive | Positive | Positive | Negative |

As shown in Table 7, it was confirmed that Examples 1 and 2 can be used to detect presence or absence of a target analyte equivalently to Comparative Examples 1 and 2.

### Test Example 2: Verification of Suitability for Quantification of a Target Analyte and Quantitative Accuracy and Precision

It was investigated whether the methods according to Examples 1 and 2 can be used for quantification of a target analyte, that is, determination of an initial amount of the target analyte.

The quantification was based on a known absolute quantification method using a standard curve of a standard material. A standard curve was obtained through real-time polymerase chain reaction using standard materials at various concentrations, and it was verified whether the methods according to Examples 1 and 2 are suitable for quantification of a target analyte based on a coefficient of determination (R²) of the standard curve and PCR efficiency. The PCR efficiency was calculated using Equation 8 below. In this test example, a cycle number of an intersection point between a first-order function passing through a first extreme point of a first-order differential function and a second extreme point of a second-order differential function and an S-shaped regression function described in Example 1 was used, and a cycle number of an intersection point between a first-order function passing through a first extreme point and a second extreme point of a second-order differential function and an S-shaped regression function described in Example 2 was used. $Efficiency \left(%\right) = \left({10}^{\frac{- 1}{Slope}}-1\right) \times 100$ Equation 8

In the above equation, slope is a slope of the standard curve.

### Test Example 2-1: Acquisition of a Standard Curve Using a Standard Material

According to Test Examples 2-1-1 to 2-1-4 below, a standard curve for absolute quantification of a target analyte was obtained.

### Test Example 2-1-1: Performance of Real-Time Polymerase Chain Reaction and Acquisition of Raw Data Sets

For each of the four concentrations of the target analyte used in Example 1-1, six repeated experiments were performed to obtain raw data sets.

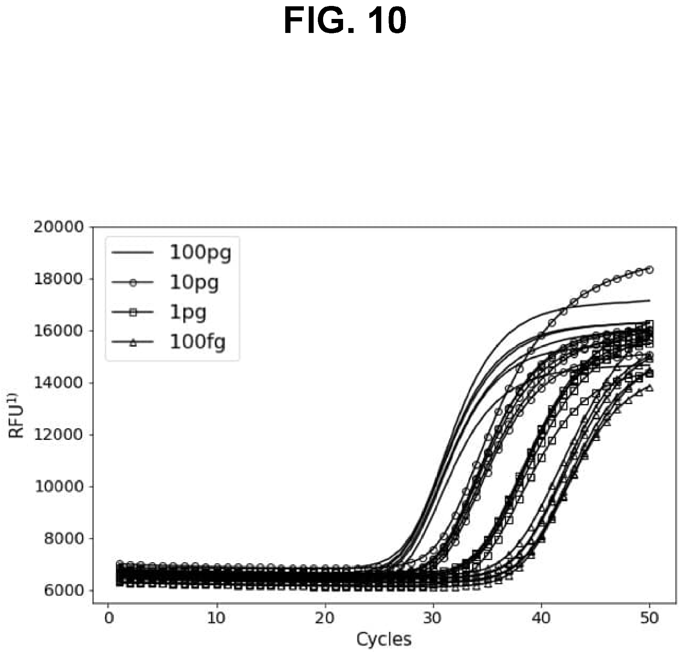

Raw data sets obtained through six repeated experiments from samples containing Neisseria gonorrhoeae (NG) genomic DNA at various concentrations as a target analyte are shown in FIG. 10.

### Test Example 2-1-2: Selection of Data Sets for Obtaining an S-Shaped Regression Function

As a result of selecting data sets using a threshold of 100 ΔRFU in the same manner as described in Example 1-3, all data sets were determined to be data sets for obtaining an S-shaped regression function.

### Test Example 2-1-3: Obtaining an S-Shaped Regression Function for a Selected Interval in Each Data Set

As described in Example 1-4, an S-shaped regression function for a selected interval in each data set was obtained, and a Richard function approximating the selected interval was determined. Values of parameters of the determined Richard functions are shown in Table 8 below.

**Table 8**

| Concentration | Replicate | | Parameter | | | | |
|---|---|---|---|---|---|---|---|
| | | Selected cycle interval | Fb | Fmax | b | c | d |
| 100 pg | 1 | | 6189 | 8421 | 3.031 | 25.53 | 5.507 |
| | 2 | | 6825 | 10243 | 2.913 | 27.02 | 3.874 |
| | 3 | | 6374 | 9033 | 2.866 | 26.95 | 3.766 |
| | 4 | | 6665 | 9578 | 2.984 | 25.76 | 5.132 |
| | 5 | | 6441 | 9526 | 3.417 | 21.92 | 12.644 |
| | 6 | | 6590 | 9669 | 3.228 | 23.39 | 9.167 |
| 10 pg | 1 | | 6319 | 8800 | 3.244 | 27.86 | 7.053 |
| | 2 | | 6598 | 9492 | 3.161 | 28.84 | 5.398 |
| | 3 | | 6549 | 9491 | 3.320 | 27.13 | 7.819 |
| | 4 | | 6510 | 9232 | 3.261 | 27.81 | 6.781 |
| | 5 | | 6386 | 9620 | 3.792 | 23.77 | 15.949 |
| | 6 | Cycles 1-50 | 6861 | 11698 | 4.164 | 21.53 | 22.309 |
| 1 pg | 1 | | 6189 | 8410 | 3.416 | 32.44 | 5.183 |
| | 2 | | 6550 | 9328 | 3.149 | 34.69 | 3.151 |
| | 3 | | 6535 | 9166 | 3.211 | 33.59 | 3.845 |
| | 4 | | 6665 | 9632 | 3.472 | 32.39 | 5.246 |
| | 5 | | 6495 | 9567 | 3.849 | 29.82 | 9.009 |
| | 6 | | 6603 | 10182 | 4.035 | 28.08 | 12.414 |
| 100 fg | 1 | | 6149 | 8302 | 3.127 | 38.47 | 3.155 |
| | 2 | | 6467 | 9085 | 2.941 | 39.47 | 2.323 |
| | 3 | | 6347 | 8650 | 2.982 | 39.51 | 2.555 |
| | 4 | | 6457 | 9267 | 3.374 | 37.80 | 3.493 |
| | 5 | | 6298 | 9278 | 3.666 | 37.14 | 4.347 |
| | 6 | | 6578 | 10812 | 4.247 | 33.30 | 7.565 |

### Test Example 2-1-4: Generation of a Standard Curve

(1) In the same manner as described in Example 1, for each Richard function, a cycle number of an intersection point between an S-shaped regression function and a first-order function passing through a first extreme point of a first-order differential function and a second extreme point of a second-order differential function was determined. Thereafter, a standard curve for use in quantification was generated by plotting log-transformed concentrations of the target analyte on an x-axis and intersection values of the first-order function of the target analyte on a y-axis.

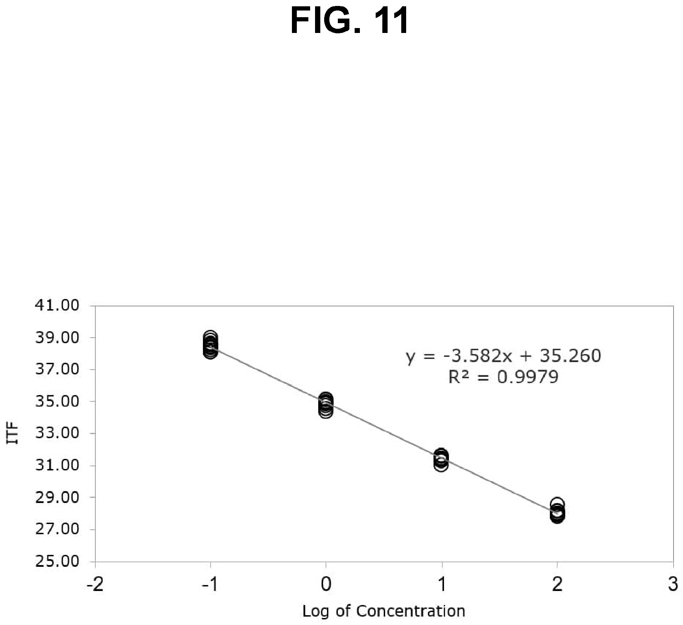

Standard curves generated using samples containing the target analyte at four different concentrations are shown in FIG. 11, and coefficients of determination (R²) and PCR efficiencies of each standard curve are shown in Table 9 below.

**Table 9**

| Replicate | Standard curve | | | |
|---|---|---|---|---|
| | Standard curve equation | R² | PCR efficiency | PCR efficiency (%) |
| 1 | y = -3.608x + 35.788 | 0.9984 | 1.893 | 89% |
| 2 | y = -3.521x + 35.786 | 0.9984 | 1.923 | 92% |
| 3 | y = -3.689x + 35.798 | 0.9941 | 1.867 | 87% |
| 4 | y = -3.553x + 35.640 | 0.9989 | 1.912 | 91% |
| 5 | y = -3.717x + 35.715 | 0.9983 | 1.946 | 95% |
| 6 | y = -3.403x + 35.665 | 0.9986 | 1.967 | 97% |
| Mean | y = -3.582x + 35.260 | 0.9979 | 1.918 | 92% |

As shown in Table 9, the average R² value of the generated standard curves was confirmed to be 0.9979, and the average PCR efficiency was confirmed to be 92%.

(2) In the same manner as described in Example 2, for each Richard function, a cycle number of an intersection point between an S-shaped regression function and a first-order function passing through a first extreme point and a second extreme point of a second-order differential function was determined. Thereafter, a standard curve for use in quantification was generated by plotting log-transformed concentrations of the target analyte on an x-axis and intersection values of the first-order function of the target analyte on a y-axis.

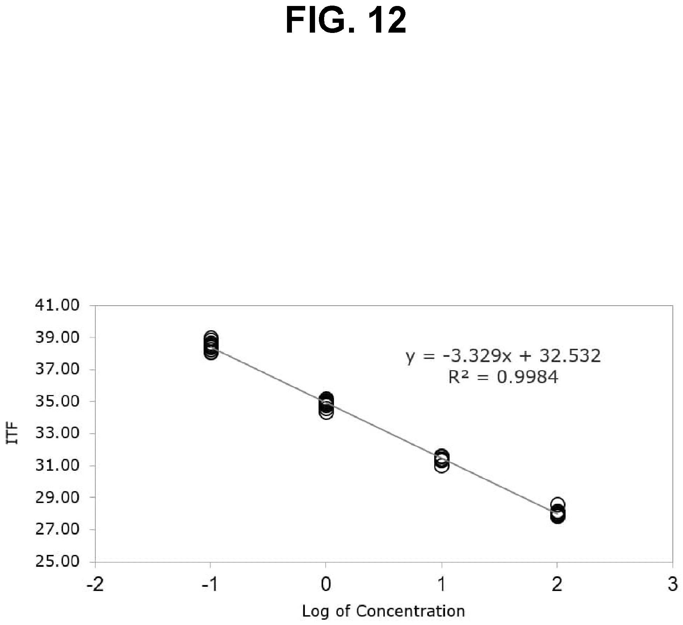

Standard curves generated using samples containing the target analyte at four different concentrations are shown in FIG. 12, and coefficients of determination (R²) and PCR efficiencies of each standard curve are shown in Table 10 below.

**Table 10**

| Replicate | Standard curve | | | |
|---|---|---|---|---|
| | Standard curve equation | R² | PCR efficiency | PCR efficiency (%) |
| 1 | y = -3.384x + 32.738 | 0.9982 | 1.975 | 97% |
| 2 | y = -3.267x + 32.536 | 0.9994 | 2.023 | 102% |
| 3 | y = -3.465x + 32.675 | 0.9955 | 1.943 | 94% |
| 4 | y = -3.275x + 32.497 | 0.9997 | 2.020 | 102% |
| 5 | y = -3.421x + 32.511 | 0.9986 | 1.960 | 96% |
| 6 | y = -3.164x + 32.209 | 0.9992 | 2.071 | 107% |
| Mean | y = -3.329x + 32.532 | 0.9984 | 1.999 | 100% |

As shown in Table 10, the average R² value of the generated standard curves was confirmed to be 0.9984, and the average PCR efficiency was confirmed to be 100%.

### Test Example 2-2: Verification of Suitability for Quantification

Suitability of the methods according to Examples 1 and 2 for quantifying a target analyte was evaluated based on R² values of standard curves and PCR efficiency.

According to the literature [2006 Bio-Rad Laboratories, Inc. *Real-Time PCR* Applications Guide], criteria for suitability of a quantification method require that an R² value corresponding to linearity of a standard curve exceed 0.980, and that PCR amplification efficiency, which theoretically increases as 2", be within a range of 90-105%.

As can be seen from Tables 9 and 10, average R² values of standard curves obtained by the methods according to Examples 1 and 2 were 0.9979 and 0.9984, respectively, both exceeding the criterion of 0.980 described in the literature, and PCR amplification efficiencies were confirmed to be within the criterion range of 90-105% described in the literature.

Accordingly, it was confirmed that the methods according to Examples 1 and 2 are suitable for quantifying a target analyte.

### Test Example 2-3: Verification of Quantitative Accuracy and Precision

Accuracy and precision of quantification of a target analyte using the methods according to Examples 1 and 2 were evaluated based on relative error (RE) values and coefficients of variation (CV).

According to the literature [2008 Introduction to Probability and Statistics], relative error represents, as a percentage, how much a predicted value differs from an actual value, and a value closer to zero indicates a smaller difference between the predicted value and the actual value. A coefficient of variation is an index representing a degree of relative variability, and a lower value indicates that data are more closely clustered around a mean.

To compare relative errors for each method, six replicated standard curves for quantification based on FDM and SDM of Comparative Examples 1 and 2 were generated using the same procedure as used in Test Example 2-1-4.

Calculation of relative error was performed using Equation 9 below, wherein copy numbers of *Neisseria gonorrhoeae* genomic DNA (100 pg, 10 pg, 1 pg, or 100 fg) used as a target analyte were used as actual values, and values back-calculated using slopes and y-intercepts of standard curves were used as predicted values. For each of the examples and comparative examples, four replicated quantification cycle values (Cq) for detection at four concentrations were generated and compared.

To compare coefficients of variation for each method, four replicated quantification cycle values (Cq) for detection at four concentrations (100 pg, 10 pg, 1 pg, or 100 fg) of *Neisseria gonorrhoeae* genomic DNA used as a target analyte were generated using the same procedure as used in Examples 1 and 2 and Comparative Examples 1 and 2.

Using the generated Cq values and Equation 10 below, coefficients of variation of the examples and comparative examples were calculated and compared, and results are summarized in Table 11 below. $Relative Error \left(%\right) = \left|\frac{Actual Value - Expected Value}{Actual Value}\right| \times 100$ Equation 9 $Coefficient of Variation \left(%\right) = \left(\frac{Standard Deviation}{Mean}\right) \times 100$ Equation 10

**Table 11**

| - | Example 1 | Example 2 | Comparative Example 1 | Comparative Example 2 |
|---|---|---|---|---|
| Mean RE | 20.28 | 5.29 | 45.95 | 20.65 |
| Mean CV | 35.27 | 37.32 | 38.32 | 38.47 |

As can be seen from Table 11, comparison of relative errors of the examples and comparative examples showed that Example 1 (20.28) and Example 2 (5.29) had smaller relative error values than Comparative Example 1 (45.95) and Comparative Example 2 (20.65), thereby confirming that Examples 1 and 2 exhibited higher accuracy. In terms of coefficients of variation, Example 1 (35.27) and Example 2 (37.32) also had smaller values than Comparative Example 1 (38.32) and Comparative Example 2 (38.47), thereby confirming that Examples 1 and 2 exhibited higher precision.

In summary, it was confirmed that the methods of Examples 1 and 2 provide improved accuracy and precision in quantifying a target analyte as compared with Comparative Examples 1 and 2.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A method for detecting a target analyte in a sample, comprising:
(a) obtaining an S-shaped function representing a growth curve by an amplification reaction for a target analyte;
(b) obtaining an nth order differential function and an n+1th order differential function from the S-shaped function; wherein the n is a natural number,
(c) obtaining a first order function connecting i) a first extreme point of the nth order differential function or the n+1th order differential function and ii) a second extreme point of the n+1th order differential function; and
(d) detecting the target analyte in the sample by using the intersection point of the S-shaped function of step (a) with the first order function of step (c).

2. A method for determining a quantification cycle (Cq) value for a target analyte in a sample, comprising:
(a) obtaining an S-shaped function representing a growth curve by an amplification reaction for a target analyte;
(b) obtaining an nth order differential function and an n+1th order differential function from the S-shaped function; wherein the n is a natural number,
(c) obtaining a first order function connecting i) a first extreme point of the nth order differential function or the n+1th order differential function and ii) a second extreme point of the n+1th order differential function; and
(d) determining the intersection point of the S-shaped function of step (a) with the first order function of step (c) as the Cq value.

3. The method according to claim 1 or 2, wherein the step of obtaining the S-shaped function in step (a) comprises the following steps:
(a-1) obtaining a first data set representing the growth curve by the amplification reaction for the target analyte; wherein the first data set includes a plurality of data points, each having a cycle number and a signal value at the cycle number;
(a-2) calculating a slope value at each cycle number for the first data set to obtain a second data set; wherein the second data set includes a plurality of data points, each having a cycle number and a slope value at the cycle number; and
(a-3) calculating the S-shaped function that approximates a selected portion of the second data set.

4. The method according to claim 3, wherein the first data set in step (a-1) is a raw data set, a mathematically modified data set of the raw data set, a normalized data set of the raw data set, or a normalized data set of the mathematically modified data set.

5. The method according to claim 3, wherein the slope value in step (a-2) is obtained by a differentiation, a difference, a difference quotient, a ratio, or linear regression of the signal values.

6. The method according to claim 3, wherein the selected portion of the second data set in step (a-3) is within a range from the first cycle number to the last cycle number in the second data set.

7. The method according to claim 3, wherein calculating the S-shaped function in step (a-3) includes determining parameters of the S-shaped function.

8. The method according to claim 1 or 2, wherein the S-shaped function in step (a) is selected from the group consisting of a sigmoid function, a logistic function, a Gompertz function, a Chapman function and a Richard function.

9. The method according to claim 1 or 2, wherein the n is a natural number selected from 1 to 10.

10. The method according to claim 1, wherein using the intersection point in step (d) includes determining the intersection point as a quantification cycle (Cq) value.

11. The method according to claim 2 or 10, wherein determining the intersection point as a quantification cycle (Cq) value includes determining a cycle number of the intersection point as the Cq value.

12. The method according to claim 3, wherein the method further comprises the following steps between step (a-1) and step (a-2):
(a-1-1) applying a threshold value to the first data set; and
(a-1-2) proceeding to the next step (a-2), if the first data set has a signal value exceeding the threshold value.

13. The method according to claim 3, wherein the method further comprises the following steps between step (a-2) and step (a-3):
(a-2-1) applying a threshold value to the second data set; and
(a-2-2) proceeding to the next step (a-3), if the second data set has a slope value exceeding the threshold value.

14. The method according to claim 1 or 2, wherein the target analyte is a target nucleic acid molecule.

15. A computer readable storage medium containing instructions to configure a processor to perform a method for detecting a target analyte in a sample, the method comprising:
(a) obtaining an S-shaped function representing a growth curve by an amplification reaction for a target analyte;
(b) obtaining an nth order differential function and an n+1th order differential function from the S-shaped function; wherein the n is a natural number,
(c) obtaining a first order function connecting i) a first extreme point of the nth order differential function or the n+1th order differential function and ii) a second extreme point of the n+1th order differential function; and
(d) detecting the target analyte in the sample by using the intersection point of the S-shaped function of step (a) with the first order function of step (c).

16. A computer readable storage medium containing instructions to configure a processor to perform a method for determining a quantification cycle (Cq) value for a target analyte in a sample, the method comprising:
(a) obtaining an S-shaped function representing a growth curve by an amplification reaction for a target analyte;
(b) obtaining an nth order differential function and an n+1th order differential function from the S-shaped function; wherein the n is a natural number,
(c) obtaining a first order function connecting i) a first extreme point of the nth order differential function or the n+1th order differential function and ii) a second extreme point of the n+1th order differential function; and
(d) determining the intersection point of the S-shaped function of step (a) with the first order function of step (c) as the Cq value.
